# EUROPEAN PATENT SPECIFICATION

(11) **EP 1 482 307 B1**
(45) Date of publication and mention of the grant of the patent: **03.10.2007**
(21) Application number: 02790949.8
(22) Date of filing: 27.12.2002
(51) Int. Cl.: C12Q 1/00, C12Q 1/60, G01N 27/327

(54) **BIOSENSOR**
BIOSENSOR
BIODETECTEUR

(30) Priority: 01.03.2002 JP 2002056190; 01.03.2002 JP 2002056191; 05.03.2002 JP 2002058992
(43) Date of publication of application: 01.12.2004
(73) Proprietor: MATSUSHITA ELECTRIC INDUSTRIAL CO., LTD, Kadoma-shi, Osaka 571-8501 (JP)
(72) Inventor: HASEGAWA, Miwa, Ako-gun, Hyogo 678-1205 (JP); YAMAMOTO, Tomohiro, Hirakata-shi, Osaka 573-0018 (JP); WATANABE, Motokazu, Toyonaka-shi, Osaka 560-0025 (JP); NAKAMINAMI, Takahiro, Toyonaka-shi, Osaka 560-0033 (JP); IKEDA, Shin, Katano-shi, Osaka 576-0022 (JP); YOSHIOKA, Toshihiko, Hirakata-shi, Osaka 573-0035 (JP); NANKAI, Shiro, Hirakata-shi, Osaka 573-0071 (JP)
(74) Representative: TBK-Patent
(86) International application number: PCT/JP2002/013875
(87) International publication number: WO 2003/074999

(56) References cited:
- WO-A-02/10734
- WO-A-96/15453
- WO-A-02/095385
- JP-A- 10 221 293
- JP-A- 2000 039 416
- JP-A- 2000 214 170
- JP-A- 2001 091 512
- JP-A- 2001 201 479
- JP-A- 2001 526 388
- JP-A- 2002 202 283
- JP-A- 2002 340 839
- LIN L ET AL: "Preparation and characterisation of novel, blood-plasma-separation membranes for use in biosensors" JOURNAL OF MEMBRANE SCIENCE, ELSEVIER SCIENCE, AMSTERDAM, NL, vol. 173, no. 1, July 2000 (2000-07), pages 73-85, XP004197648 ISSN: 0376-7388

## Description

### Technical Field

The present invention relates to a biosensor capable of carrying out rapid, highly-sensitive, simple determination of a specific component in a sample to be detected such as blood, serum and plasma, especially a biosensor capable of measuring glucose, total cholesterol and the like.

### Background Art

A description is given to an example of a conventional biosensor in terms of a glucose sensor. As a typical example, there is a glucose sensor obtained by forming an electrode system including at least a measuring electrode and a counter electrode on an insulating base plate by a method such as screen printing and then forming an enzyme reaction layer including a hydrophilic polymer, oxidoreductase and an electron mediator on the electrode system. As oxidoreductase used is glucose oxidase; as the electron mediator used is a metal complex, an organic compound or the like, such as potassium ferricyanide, ferrocene derivative or quinone derivative. A buffer is added to the enzyme reaction layer as required.

When a sample solution containing a substrate is added dropwise onto the enzyme reaction layer in this biosensor, the enzyme reaction layer dissolves to cause a reaction of the enzyme with the substrate, which accompanies reduction of the electron mediator. After completion of the enzyme reaction, the substrate concentration in the sample solution can be determined from a value of an oxidation current, which is obtained when the reduced electron mediator is electrochemically oxidized.

In this type of glucose sensor, a reductant of the electron mediator generated as a result of the enzyme reaction is oxidized at the electrode, and the glucose concentration is determined from the oxidation current value.

Such a biosensor is theoretically capable of measuring diverse substances by using an enzyme whose substrate is an object to be measured. For example, when cholesterol oxidase or cholesterol dehydrogenase is used as oxidoreductase, it is possible to measure a cholesterol value in a serum to be used as a diagnostic indicator in various medical institutions.

Because the enzyme reaction of cholesterol esterase proceeds very slowly, with an appropriate surfactant added thereto, the activity of cholesterol esterase can be improved to reduce the time required for the overall reaction.

However, the surfactant, as being included in the reaction system, has an adverse effect on hemocytes, making it impossible to measure whole blood itself, as done in the glucose sensor.

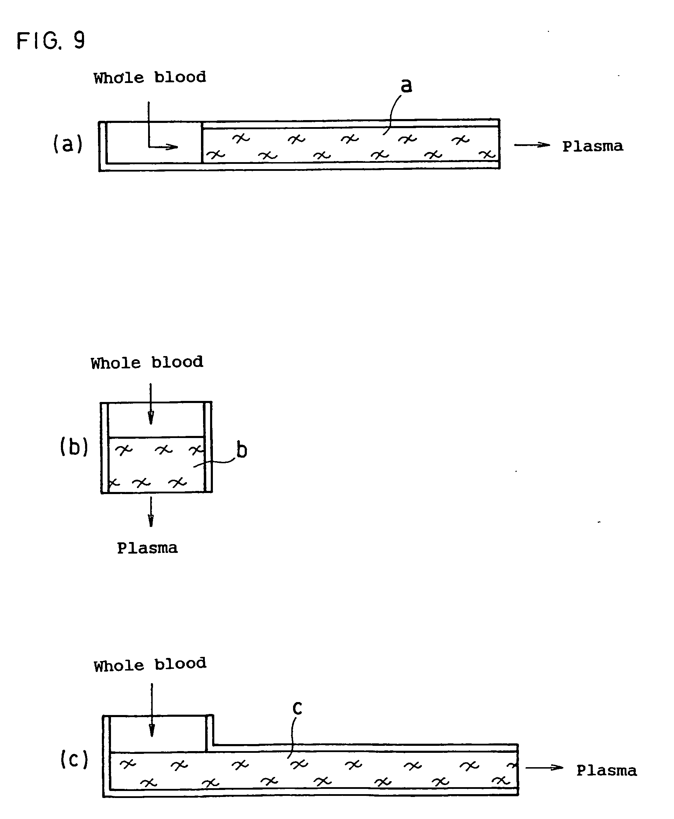

Thereat, a proposal has been made to provide a filter (hemocyte-filtering part) in the vicinity of an inlet of a sample solution supply pathway for rapid supply of only plasma with hemocytes therein filtered, into a sensor. FIG. 9 shows a schematic sectional view of a filter device for use in explanation of a mechanism for separating blood.

There are three types of methods for separating blood as follows:

### · Lateral Separation Method:

As shown in (a) in FIG. 9, blood is dropped onto the side end of the sample solution supply part side portion of a filter "a", which is filtered in the lateral direction to exude plasma from the end of the air aperture side portion of the sample solution supply pathway of the filter "a".

### · Vertical Separation Method:

As shown in (b) in FIG. 9, blood is dropped directly onto the upper face of a filter "b", which is filtered in the vertical direction to exude plasma from the end of the bottom or the vicinity thereof of the filter "b".

### · Combination Separation Method:

As shown in (c) in FIG. 9, blood is dropped directly onto the upper face of a sample solution supply pathway side portion of a filter "c", which is filtered in the vertical direction, as well as in the lateral direction, to exude plasma from the end of the air aperture side portion of the sample solution supply pathway of the filter "c".

The type conventionally in general use is the lateral separation (e.g. Japanese Patent Application No. 2000-399056) or the combination separation (e.g. Japanese Patent Application No. 2001-152868).

When the filter is inappropriately incorporated in the sensor, however, hemocytes captured in the filter are destroyed and hemoglobin dissolves out. As the hemocytes are destroyed and become small components of about the size of the hemoglobin, it becomes difficult to filter such small components with the filter. Hence the hemoglobin flows into the sample solution supply pathway, which may cause a measurement error.

This is presumably caused by the fact that a difference in thickness between the filter before absorbing a sample solution and the expanded filter after absorbing the sample solution is not fitted to a gap between pressing parts for holding the filter from the top and the bottom. When the gap between the pressing parts for holding the filter from the top and the bottom is too narrow for the thickness of the expanded filter, the filter is prevented from expanding. The pore size of the filter thus prevented from expanding cannot widen sufficiently, to destroy hemocytes infiltrating thereinto.

As opposed to this, when the gap between the upper and lower pressing parts is previously set wide for the supposed thickness of the expanded filter, the filter may slide during preservation because hematocrit values (ratios of red cell volume) are different depending on sample solutions, leading to different degrees of expansion of the filter.

In order to solve this problem, it has been considered that in the combination separation method, the pressing part for holding the filter surface is brought into contact with any portion of either the top or the bottom of the filter (e.g. Japanese Patent Application No. 2001-152868).

With this configuration, even when the distance between the upper pressing part and the lower pressing part for holding the filter is not fitted to the thickness of the filter expanded as absorbing the sample solution, it is possible to prevent expansion of the filter from being inhibited and further to avoid a measurement error caused by the hemocyte destruction due to the inhibition of the expansion.

That is to say, since the pressing parts for holding the filter are not holding the filter from the top and the bottom, the filter can expand in the space portion and in the sample solution supply part except the pressing part so that the pore size of the filter can be freely changed and no hemocyte destruction will occur.

While being effective as methods for reducing an amount of a sample solution, these methods have a problem that the structure of a biosensor thus obtained becomes complex. In a case of using a flat filter in triangle shape in a vertically-shaded view, for example, it has been very difficult in terms of production to insert the peak portion of the head thereof into the inlet (width 0.8 mm) of the sample solution supply pathway even for about 1 mm.

There has also been a problem in the lateral separation method as well as the combination separation method that the flow rate of the filtrate is lower than that in the vertical separation method. Although it is possible to simplify the structure of the obtained biosensor to deal with this problem, it has been structurally impossible to employ the normal vertical separation method because of the need for carrying a reagent in a portion opposed to the electrode when the biosensor is applied as a cholesterol sensor. This is by reason of a structural problem and a problem regarding the position of carrying the reagent.

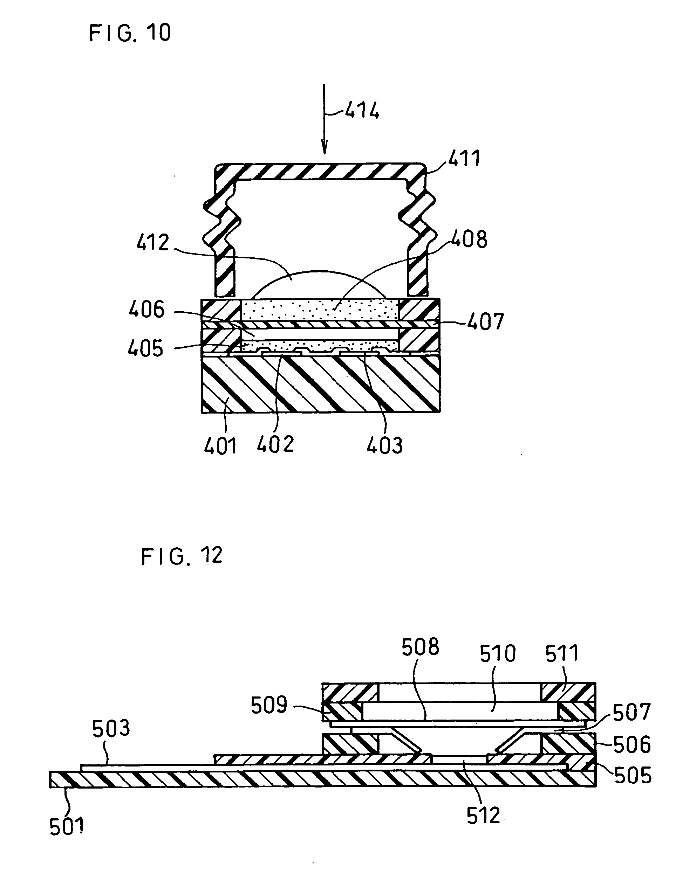

Firstly, when the vertical separation method, pointed out in Japanese Patent Application No. Sho 62-180434 and Japanese Patent Application No. Sho 62-292323, is used, for example, there has been required employment of the structures shown in FIGS. 10 to 12.

In the biosensor of the type shown in FIG. 10, a working electrode 402 and a counter electrode 403 are provided on an insulating base plate 401, on which an oxidoreductase layer 405, a space 406, a filter 407 and a porous plate 408 are provided. The filtration at this time is classified as a natural filtrating method utilizing gravity, but the flow rate thereat is slow since bubbles may be generated in the sample solution or the sample solution is resistant to getting into the porous plate 408. This may result in arrival of an insufficient amount of the filtrate at the electrode, raising a problem of poor measurement accuracy. To this end, a pressure device 411 of a pump type is provided in the upper part and pressure 414 is applied to filter the sample solution 412.

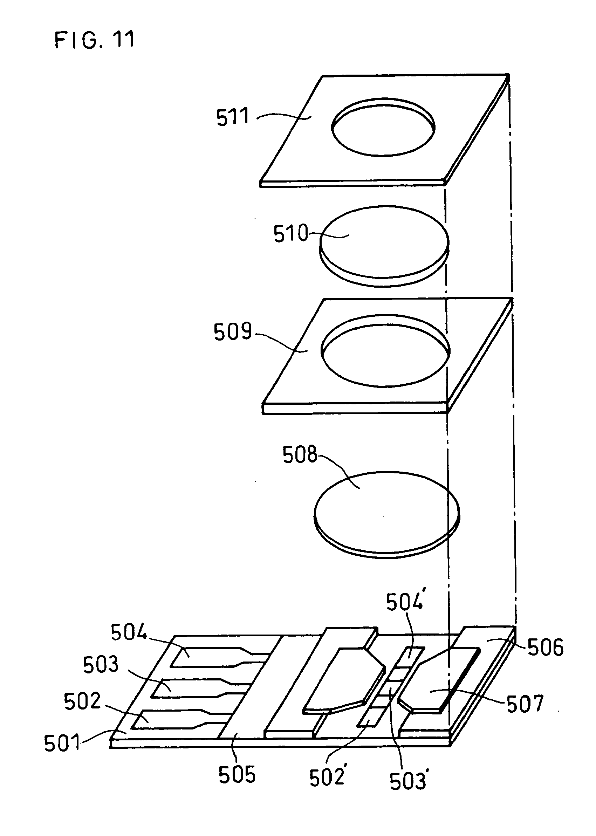

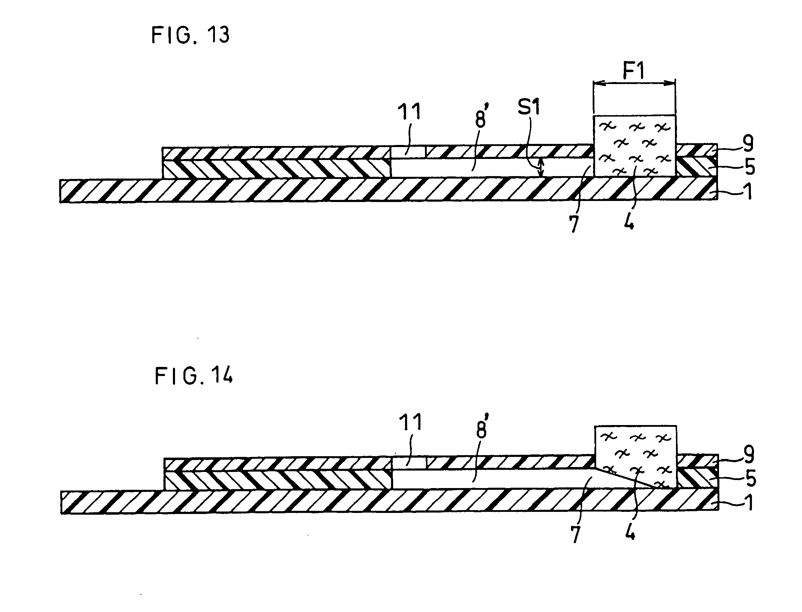

In this respect, in the sensor described in Japanese Patent Application Sho No. 62-292323, an inducing layer 507 is disposed into the sensor, for leading the filtrate having passed through a filter 508 to electrodes 502', 503' and 504', in place of the pressure device, as shown in FIGS. 11 and 12. In this arrangement, the filtrate is led by the inducing layer 507 to get the electrode wet first and then expands over the electrode with the aid of a hydrophilic polymer layer 512, enabling highly-accurate measurement without generation of bubbles on the working electrode.

It should be noted that in FIGS. 11 and 12, a filter 508, a holding frame 509, a porous plate 510 and a cover 511 are provided on the upper part of the inducing layer 507. Electrodes 502, 503 and 504 are disposed on the upper part of a base plate 501 and an insulating layer 505 is provided thereon.

However, since a pressure device, an inducing layer and the like are required in any case, a problem may arise that the sensor is structurally complex.

Further, a problem regarding the position of carrying the reagent is described in Japanese Patent Application No. 2000-018834. In the cholesterol sensor disclosed in this specification, a reagent with a very high concentration is carried, unlike a blood sugar sensor. When a necessary reagent is carried in one place as in the blood sugar sensor, therefore, a problem may arise that dispersion of the reagent is prevented to result in lower accuracy of the response current value. Further, since a surfactant is contained in the reagent, which is not present in the blood sugar sensor, to exert an adverse effect on preservation stability of other reagents, the reagent needs to be separated for being carried. It has therefore been impossible to employ the conventional structure that the reagent is separated above and below (to the electrode and to the cover side) to be carried and the filter is placed on the upper part of the electrode.

The document WO 02/10734 A discloses a biosensor comprising an insulating base plate; an electrode system, provided on the base plate, having a measuring electrode and a counter electrode; a reaction layer comprising at least an oxidoreductase and an electron mediator; a sample solution supply pathway including the electrode system and the reaction layer; and a sample supply unit, wherein the sensor is so structured that, between the sample supply unit and the sample solution supply pathway. There is provided a filter having a function to filter out hemocytes and having a cross-sectional area larger than an opening of the sample solution supply pathway, and that plasma of a blood with hemocytes thereof having been filtered out is sucked into inside of the sample solution supply pathway owing to capillary phenomena (cf. abstract). Moreover, an air vent 22 is disclosed (cf. [0022]), as well as an opening portion 18 is provided.

The document WO 02/095385 A describes a cholesterol sensor with high-accuracy and excellent response, whose object to be measured is whole blood, where plasma with hemocytes therein filtered can rapidly reach an electrode system. In a biosensor where plasma with hemocytes therein filtered with a filter is sucked into a sample solution supply pathway due to capillarity, there are formed: a first pressing part for holding a primary side portion of the filter from the bottom; a second pressing part for holding a secondary side portion of the filter from the top and the bottom; a third pressing part for holding the central portion of the filter from the top; and a void for surrounding the filter between the second pressing part and third pressing part.

Accordingly, a first objective of the present invention is to provide a biosensor improved such that the aforesaid disadvantage is avoided when the vertical separation method is employed and plasma obtained as hemocytes are separated by the filtration of blood promptly reaches the electrode system.

Secondary, in any filtrating method of the lateral separation method (e.g. Japanese Patent Application No. 2000-236131, Japanese Patent Application No. 2000-399056 and Japanese Patent Application No. 2001-152868), the vertical separation method (e.g. Japanese Patent Application No. 2001-180362) and the combination separation method, there may be cases where hemocytes captured in the filter are destroyed and hemoglobin elutes when a filter is not suitable. It is difficult to filter small hemocyte components of about the size of hemoglobin, and hemoglobin flows into the sample solution supply pathway, which may cause a measurement error.

There has in particular been a problem, especially in the case of the lateral separation method, that the sensor structure may become complex, and there has been a common problem of frequent occurrence of a measurement error attributed to the mixing of the hemocytes. This is because a reservation ratio of a conventional glass fiber filter paper (depth filter) is 98% due to the characteristic thereof, and 2% thereof is thus flown out. There has further been a problem, even with a reduced sensor volume, that whole blood is absorbed into a filter constituted by thick glass fiber filter paper (e.g. thickness 200 to 800 *µ*m), whereby reduction in sample amount is limited.

It is therefore a second object of the present invention to obviate the aforesaid disadvantages and provide a biosensor improved such that filtered plasma is moved to the sample solution supply pathway due to capillary action without pressure and the filtrate stops at the air aperture while no hemocyte is mixed. Further, it is an object of the present invention to obtain a biosensor, where, especially in the case of measurement based on collection of blood by puncturing a fingertip, whole blood on the fingertip is effectively rubbed against the sensor with ease and plasma as an object to be measured can be rapidly supplied to the electrode system.

Thirdly, the conventional examples regarding the separation of blood by the use of two types or more of filters are described, for example, in US Patent No. 5,240,862 (Applicants: X-Flor B.V. and Primecare B.V.), WO Publication No. 96/15453 (Applicant: Spectral Diagnostic Inc.) and the like. It is characterized here that a sample solution is passed successively from a filter with a larger pore size to a filter with a smaller pore size. In other words, it is characterized in that a sample solution passes successively from a filter with a larger pore size to a filter with a smaller pore size as it flows from the inlet to the outlet.

In the technique using two types or more of filters as thus described, however, in the case of arranging a filter with a 100% capture rate, which will not get hemocytes to pass therethrough, on the lowest layer, there has been a problem that a small amount of a filtrate obtained from the air aperture side portion of the sample solution supply pathway cannot be moved to a hollow sample solution supply pathway by means of natural dropping such as gravity, without applying pressure. Namely, there has been a problem that the filtrate cannot be introduced into the sample solution supply pathway due to capillary action.

It is therefore a third object of the present invention to obviate the aforesaid disadvantages and provide a biosensor improved such that a filter with such a small size as not getting hemocytes to pass therethrough is used as a first filter, an obtained filtrate is moved to the sample solution supply pathway due to capillary action without applying pressure and the filtrate stops at the air aperture. More specifically, it is an object of the present invention to provide a glucose sensor and a cholesterol sensor which have high accuracy and excellent response and whose object to be measured is whole blood.

### Disclosure of Invention

In order to achieve the aforesaid objectives, the present invention provides a biosensor comprising: an insulating base plate; an electrode system having a working electrode and a counter electrode which are provided on the base plate; a reaction layer including at least oxidoreductase and an electron mediator; a sample solution supply pathway which includes the electrode system and the reaction layer and has an inlet and an air aperture; a sample solution supply part for introducing a sample solution, which is in position apart from the sample solution supply pathway; and a first filter which is disposed between the sample solution supply pathway and the sample solution supply part for filtering the sample solution, where a filtrate filtered with the first filter is supplied into the sample solution supply pathway due to capillary action, characterized in that the direction in which the sample solution passes through the first filter and the direction in which the filtrate passes through the sample solution supply pathway cross at right angles, characterised in that said first filter does not intrude into said sample solution supply pathway.

It is preferable that the first filter is constituted by either a glass fiber or a cellulose fiber.

It is also preferable that the first filter is not in contact with the electrode system.

It is also preferable that at least part of the first filter is in contact with the insulating base plate.

It is preferable that the first filter is coated with any of polyvinyl alcohol, ethyl cellulose, hydroxypropyl cellulose, carboxymethyl cellulose, polyvinyl pyrrolidone, gelatin, agarose, polyacrylic acid and the salts thereof, starch and the derivatives thereof, polymers of maleic anhydride and the salts thereof, polyacrylamide, methacrylate resin, and poly-2-hydroxyethyl methacrylate.

It is preferable that the reaction layer includes a reagent system for detecting total cholesterol.

It is effective here that the first filter is cylindrical. It is effective at this time that the circular end face of the first filter has a diameter of not more than 5 mm.

It is also effective that an inlet is provided in the upper part of the first filter, for dropping the sample solution.

It is also effective that an aperture toward the first filter is provided in the bottom of the sample solution supply part. It is further effective that the size of the inlet of the sample solution supply part is smaller than the size of the first filter. It is effective that there is arranged a portion on the surface of the first filter, which is not in contact with other constituents of the biosensor.

It is preferable in the biosensor in accordance with the present invention that the first filter is constituted by a filter with a uniform pore size in the form of a membrane.

It is also preferable that a hydrophilic layer is provided between the first filter and the base plate.

It is also preferable that a second filter is further provided between the first filter and the sample solution supply pathway.

It is also preferable that the mean pore size of the first filter is smaller than the mean pore size of the second filter.

It is preferable that the second filter is a depth filter.

The first filter may be in contact with the second filter.

Further, the second filter may be in contact with the inlet of the sample solution supply pathway. It is preferable that the second filter is not in contact with the electrode system.

It is also preferable that the first filter and/or the second filter contain a reagent for suppressing a reaction between oxidoreductase and cholesterol contained in any of high density lipoprotein, low density lipoprotein and very low density lipoprotein in the sample solution.

### Brief Description of Drawings

FIG. 1 is an exploded perspective view of a biosensor in accordance with one embodiment of the present invention.
FIG. 2 is a combined perspective view of the biosensor in FIG. 1.
FIG. 3 is a sectional view of the main part taken on the line X-X of FIG. 2, with a reaction layer and the like omitted.
FIG. 4 is a sectional view of the main part taken on the line X-X of FIG. 2, with the reaction layer shown specifically.
Fig. 5 is an exploded perspective view of a biosensor in accordance with one embodiment of the present invention.
FIG. 6 is a combined perspective view of the biosensor in FIG. 5.
FIG. 7 is a sectional view of the main part taken on the line Y-Y of FIG. 6, with a reaction layer and the like omitted.
FIG. 8 is a sectional view of the main part taken on the line Y-Y of FIG. 6, with the reaction layer shown specifically.
FIG. 9 is a schematic sectional view of a filter device for explaining the mechanism of separating blood.
FIG. 10 is a vertical sectional view of a conventional biosensor.
FIG. 11 is an exploded perspective view of another conventional biosensor.
FIG. 12 is a vertical sectional view of the biosensor shown in FIG. 11.
FIG. 13 is a sectional view of the main part specifically showing a first filter portion of a biosensor in accordance with one embodiment of the present invention.
FIG. 14 is a sectional view of the main part specifically showing a first filter portion of a biosensor in accordance with another embodiment of the present invention.
FIG. 15 is a sectional view of the main part specifically showing a first filter portion of a biosensor in accordance with still another embodiment of the present invention.
FIG. 16 is a graph showing a response characteristic of a biosensor in Example 1.
FIG. 17 is a graph showing a response characteristic of a biosensor in Example 2.
Fig. 18 is an exploded perspective view of a biosensor in accordance with one embodiment of the present invention.
FIG. 19 is a combined perspective view of the biosensor in FIG. 18.
FIG. 20 is a vertical sectional view of the biosensor taken on the line X'-X' shown in FIG. 19, with a reaction layer and the like omitted.
FIG. 21 is a vertical sectional view of the biosensor taken on the line X'-X' shown in FIG. 19, with the reaction layer shown specifically.
FIG. 22 is a graph showing the relationship between the total cholesterol concentration in the sample solution and the current response value in Example 1 of the present invention.
FIG. 23 is a graph showing the relationship between the glucose concentration in the sample solution and the current response value in Example 2 of the present invention.
Fig. 24 is an exploded perspective view of a biosensor in accordance with one embodiment of the present invention.
FIG. 25 is a combined perspective view of the biosensor in FIG. 24.
FIG. 26 is a vertical sectional view of the biosensor taken on the line X''-X'' shown in FIG. 25, with a reaction layer, an electrode system and the like omitted.
FIG. 27 is a vertical sectional view of the biosensor taken on the line X''-X'' shown in FIG. 25, with the reaction layer, the electrode system and the like shown specifically.
FIG. 28 is a graph showing the response characteristic of the biosensor with respect to the total cholesterol in Example 5 of the present invention.

### Best Mode for Carrying Out the Invention

The present invention relates to a biosensor comprising: an insulating base plate; an electrode system having a working electrode and a counter electrode which are provided on the base plate; a reaction layer including at least oxidoreductase and an electron mediator; a sample solution supply pathway which includes the electrode system and the reaction layer and has an inlet and an air aperture; a sample solution supply part for introducing a sample solution, which is in position apart from the sample solution supply pathway; and a first filter which is disposed between the sample solution supply pathway and the sample solution supply part for filtering the sample solution, where a filtrate filtered with the first filter is supplied into the sample solution supply pathway due to capillary action, characterized in that the direction in which the sample solution passes through the first filter and the direction in which the filtrate passes through the sample solution supply pathway cross at right angles, characterised in that said first filter does not intrude into said sample solution supply pathway.

The obtained biosensor can be simplified in terms of the structure thereof by comprising these technical matters. It is further possible in this biosensor to avoid disadvantages in employing the vertical separation method and to make plasma, obtained by separating hemocytes by means of filtration of blood, rapidly arrive at the electrode system.

It is also possible that the filtered plasma is moved to the sample solution supply pathway due to capillary action without pressure and the filtrate arrives at the air aperture while no hemocyte is mixed. More specifically, it is possible to apply the biosensor in accordance with the present invention to a glucose sensor, a cholesterol sensor and the like, which have high accuracy and excellent response and whose object to be measured is whole blood.

The electron mediator for use in the present invention can be selected from potassium ferricyanide or a redox compound having the electron transferring ability to and from oxidoreductase such as cholesterol oxidase.

Oxidoreductase is an enzyme whose substrate is an object to be measured, and glucose oxidase is applied to a sensor where glucose is the object to be measured. For measurement of a cholesterol value in a serum to be used as a diagnostic indicator, cholesterol oxidase or cholesterol dehydrogenase which is an enzyme for catalyzing an oxidation reaction of cholesterol, and cholesterol esterase which is an enzyme for catalyzing the process of changing cholesterol ester to cholesterol are used. Because the enzyme reaction of cholesterol esterase proceeds very slowly, with an appropriate surfactant added thereto, the activity of cholesterol esterase can be improved to reduce the time required for the overall reaction.

The reaction layer including reagents such as the electron mediator and oxidoreductase are disposed on or in the vicinity of the electrode system in the sensor. In a sensor which comprises a cover member, to be combined with the base plate with the electrode system disposed thereon, for forming the sample solution supply pathway for supply of the sample solution to the electrode system between the base plate and the sensor, the reaction layer can be provided in the portion exposed to the sample solution supply pathway, the inlet of the sample solution supply pathway, or the like. Namely, the reaction layer can be provided on either the insulating base plate or the cover member so long as it is within the sample solution supply pathway. Wherever the place is, it is preferable that the sample solution introduced can dissolve the reaction layer with ease and arrive at the electrode system. It is also preferable to form the hydrophilic polymer layer in contact with the upper face of the electrode system so as to protect the electrode and prevent the reaction layer formed from being peeled off. Besides the electrode system, it is preferable that the hydrophilic polymer layer is formed as the base of the reaction layer as formed or the hydrophilic polymer is included in the lowest layer when the reaction layer is constituted by plural layers.

It is preferable that the reaction layer including the electron mediator is separated from the surfactant for enhancing the solubility. It is also preferable that it is separated from enzyme cholesterol oxidase and cholesterol esterase, which catalyze the oxidation reaction of cholesterol, for the sake of preservation stability.

With respect to a biosensor for measuring a blood sugar level, there is an example where a layer including lipid is formed so as to coat a layer formed on the electrode system, or the like, to facilitate introduction of the sample solution to the reaction layer (e.g. Japanese Unexamined Patent Publication No. Hei 2-062952). In the biosensor for measuring cholesterol of the present invention, it is preferable to form part of the reaction layer by freeze-drying (e.g. Japanese Patent Application No. 2000-018834) or to process the surface of a cover member to become hydrophilic by means of a surfactant, plasma irradiation or the like. Application of such a configuration can eliminate the need for a lipid layer.

The examples of the hydrophilic polymer may include, in addition to water-soluble cellulose derivatives such as ethyl cellulose, hydroxypropyl cellulose and carboxymethyl cellulose, polyvinyl pyrrolidone, polyvinyl alcohol, gelatin, agarose, polyacrylic acid and the salts thereof, starch and the derivatives thereof, polymers of maleic anhydride or the salts thereof, polyacrylamide, methacrylate resin, and poly-2-hydroxyethyl methacrylate.

The examples of the surfactant may include n-octyl-*β*-D-thioglucoside, polyethylene glycol monododecyl ether, sodium cholate, dodecyl-*β*-maltoside, sucrose monolaurate, sodium deoxycholate, sodium taurodeoxycholate, N,N-bis (3-D-gluconeamidopropyl) deoxycholeamide and polyoxyethylene (10) octyl phenyl ether. One except them as the surfactant may be used in the region that the effect of the present invention is not weakened.

When the lipid is used, favorably used for example is an amphipathic phospholipid such as lecithin, phosphatidyl choline or phosphatidyl ethanolamine.

As the measuring method of the oxidation current, a two-electrode system composed only of a measuring electrode and a counter electrode and a three-electrode system further comprising a reference electrode are applicable. The two-electrode system is more advantageous in terms of cost as well as simplification of the sensor structure, while the three-electrode system is more advantageous when more accurate measurement is required.

In the following, the present invention is described in detail with the use of concrete embodiments.

### Embodiment 1

A biosensor in accordance with Embodiment 1 of the present invention comprises: an insulating base plate; an electrode system having a working electrode and a counter electrode which are provided on the base plate; a reaction layer including at least oxidoreductase and an electron mediator; a sample solution supply pathway which includes the electrode system and the reaction layer and has an air aperture on the end side; a sample solution supply part for introducing a sample solution; and a first filter which is disposed between the sample solution supply pathway and the sample solution supply part for filtering the sample solution, where a filtrate filtered with the first filter is absorbed into the sample solution supply pathway due to capillary action. This biosensor is characterized in that the first filter does not intrude into the sample solution supply pathway, the direction in which the sample solution passes through the first filter is vertical, and further, the direction in which the plasma passes from the inlet of the sample solution supply pathway toward the air aperture thereof is lateral.

In the biosensor in accordance with Embodiment 1 of the present invention, the vertical separation method is employed as the method for separating hemocytes, in which blood is used as the sample solution, and plasma which has exuded from the inlet side of the sample solution supply pathway of the first filter flows through the sample solution supply pathway laterally from the inlet toward the air aperture at the end thereof while gradually dissolving the reagent. It is thereby possible to simplify the sensor structure without changing the position of the reagent separated and carried above and below in the sample solution supply pathway.

Further, with the first filter not intruding into the sample solution supply pathway, the filter does not come in contact with a reagent included in the reaction layer in the sample solution supply pathway and the reagent is thus difficult to diffuse in the filter. This prevents variation of the reagent concentration, allowing realization of stable sensor accuracy.

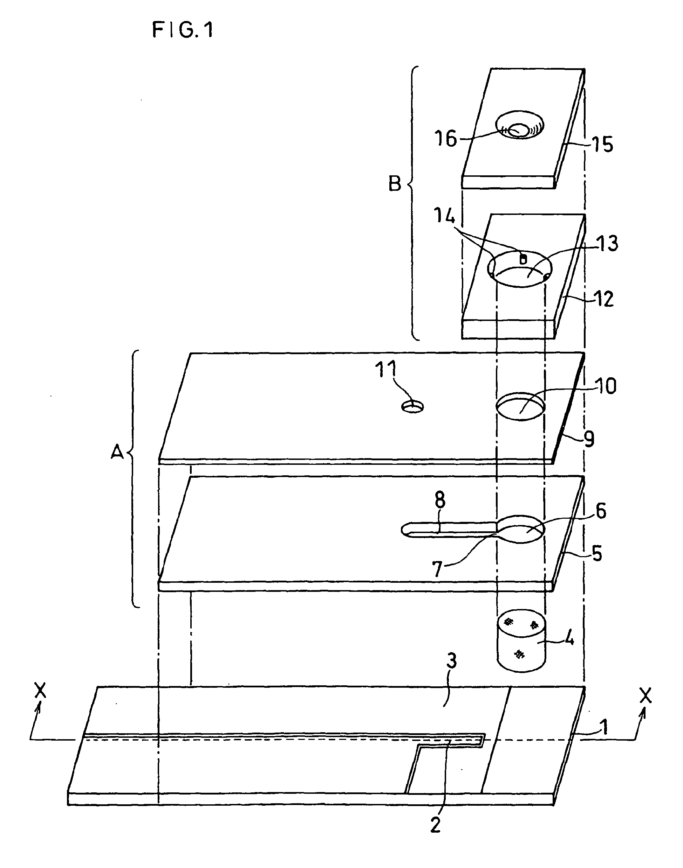

Herein, FIG. 1 is an exploded perspective view of the biosensor in accordance with Embodiment 1. As indicated in FIG. 1, the biosensor in accordance with the present invention has an insulating base plate 1 made of an insulating resin such as polyethylene terephthalate. In FIG. 1, on the left upper face of the base plate 1, a palladium portion is formed by means of sputtering, vapor deposition or the like, followed by laser trimming, to form an electrode system including a working electrode 2 and a counter electrode 3. The area of the electrode is determined corresponding to a width of a slit 8 formed on a spacer 5, as later described.

In the spacer 5 to be combined with the base plate 1 formed are the slit 8 for forming the sample solution supply pathway, an inlet 7 of the slit 8 and an aperture 6 for accommodating the first filter 4, in the obtained biosensor. Further, in a cover 9 formed is an air aperture 11 in addition to a communicating part 10 for accommodating the first filter 4.

In a filter holding plate 12 formed are a space 13 and a protruding filter holding part (protruding part) 14 for directly supporting the first filter 4. Moreover, an opening 16 communicating to the upper surface of the first filter 4 is formed in an upper cover 15 constituting the sample solution dropping part. It is to be noted that the filter holding plate 12 may be comprised of plural members, as indicated in Example 2 later described.

In integration of the aforesaid base plate 1, spacer 5, cover 9, filter holding plate 12 and upper cover 15, the aperture 6 leading to the slit 8, the aperture 10 formed in the cover 9, the space 13 formed in the filter holding plate 12 and the opening 16 formed in the upper cover 15 are communicated.

The first filter 4 as a hemocyte-filtering part is made of glass fiber filter paper, and has the shape of a cylinder with a pore size of 0.5 to 5 *µ*m, a diameter of 2 to 5 mm and a height (thickness) of 200 to 1,000 *µ*m. It should be noted that the pore size of the filter refers to a particle size when 98% of the number of particles are held in the filter. When particles with a particle size of 5 *µ*m is filtered with the use of a filter with a pore size of 5 *µ*m, the ratio of the particles held in the filter is 98%.

For assembly of this sensor, a reaction layer is formed on a prescribed portion of the base plate 1 as subsequently described. Further, based on the positional relationship shown in FIG. 1, in a concave portion (sample solution supply pathway) formed by the slit 8 of the combined base plate A obtained by combination of the cover 9 and the spacer 5, a reaction layer is formed on a prescribed member as later described. Further, based on the positional relationship shown in FIG. 1, a combined base plate B obtained by combination of the filter holding plate 12 and the upper cover 15 is prepared. Subsequently, the base plate 1, the combined base plate A, the first filter 4 and the combined base plate B are disposed as shown by the dotted line and the dashed lines in FIG. 1.

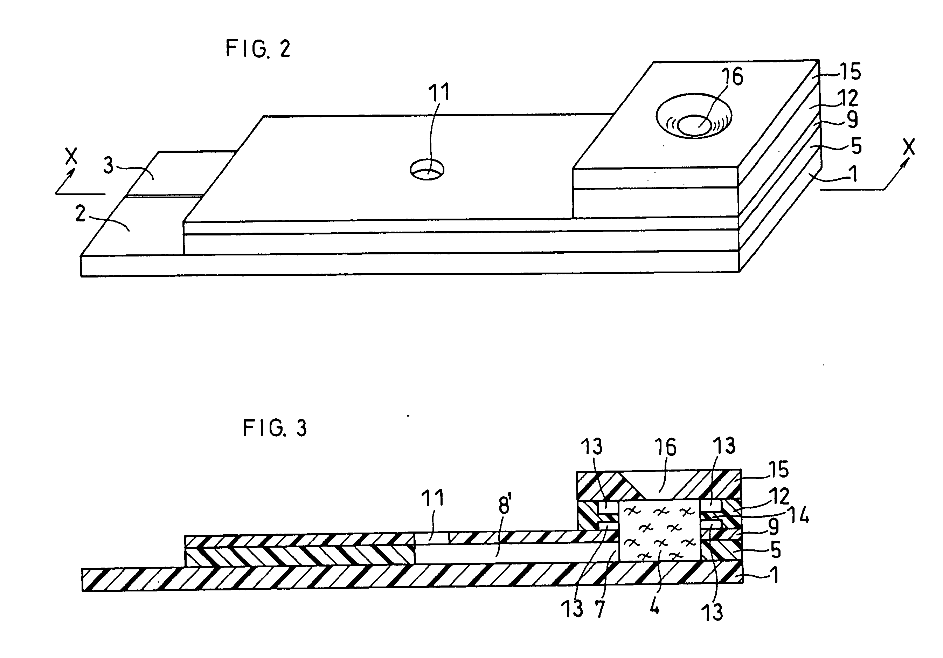

A schematic perspective view of the biosensor in accordance with the present invention obtained according to the configuration of FIG. 1 is shown in FIG. 2. A sectional view taken on the line x-x of FIG. 2 is further shown in FIG. 3. As shown in the sectional view in FIG. 3, in the biosensor in accordance with the present invention formed is the space 13 for getting the first filter 4 out of contact with the other members. For complete removal of hemocytes as interfering substances, it is necessary to provide at least one place not in contact with the filter holding part in the region from the opening 16 to the inlet 7, namely the space 13 surrounding the filter surface, in order to get the sample solution to certainly pass through the filter.

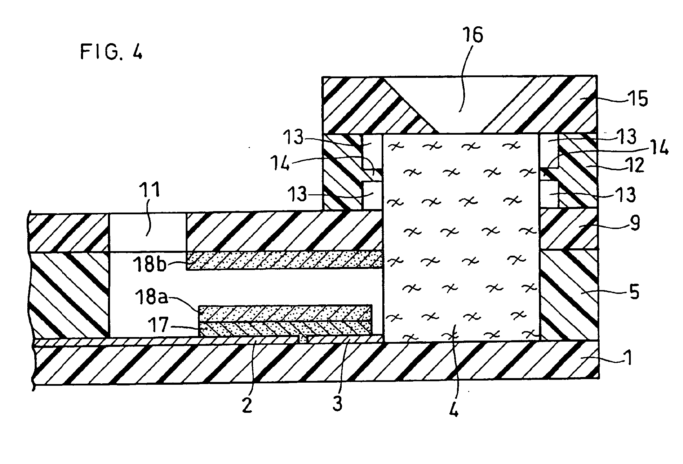

The reaction layer and the electrode system are omitted from FIG. 2 whereas a partially enlarged sectional view corresponding to FIG. 3 which represents the reaction layer and the electrode system is shown in FIG. 4. On the electrodes 2 and 3 of the base plate 1 formed are a hydrophilic polymer layer 17 and a reaction layer 18a.
Further, a reaction layer 18b is formed on the lower face of the cover 9 corresponding to the ceiling of the sample solution supply pathway. It should be noted that the other members shown in FIG. 4 are equivalent to those shown in FIG. 3.

The biosensors shown in FIGS. 1 to 4 are produced using the filter 4 and five types of members (base plates) so as to make the structures thereof easy to understand. However, the upper cover 15 and the filter holding plate 12 may be formed by one member, or the cover 9 may be further added thereto so that the whole is formed by one member. Moreover, the filter holding plate 12 or the filter holding part 14 may be omitted depending on the thickness of the first filter 4.

### Embodiment 2

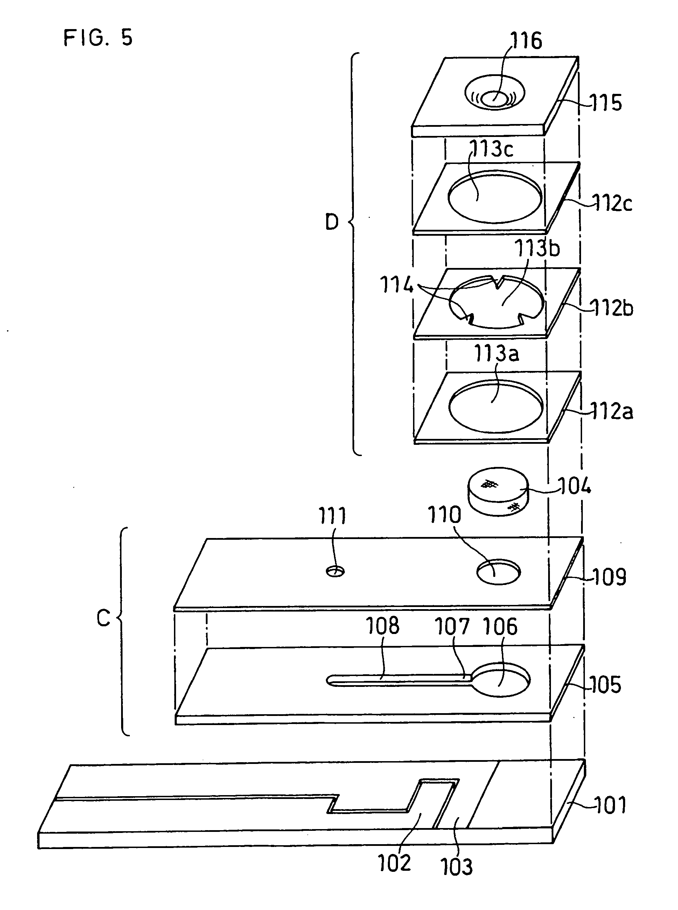

FIG. 5 is an exploded perspective view of a biosensor in accordance with Embodiment 2 obtained by further improving the biosensor in accordance with Embodiment 1 above. As shown in FIG. 5, the biosensor in accordance with the present invention has an insulating base plate 101 made of an insulating resin such as polyethylene terephthalate. In FIG. 5, on the left upper face of the base plate 101, a palladium portion is formed by means of sputtering, vapor deposition or the like, followed by laser trimming, to form an electrode system including a working electrode 102 and a counter electrode 103. The area of the electrode is determined corresponding to a width of a slit 108 formed on a spacer 105, as later described.

In the spacer 105 to be combined with the base plate 101 formed are the slit 108 for constituting the sample solution supply pathway, in the biosensor obtained after being assembled, an inlet 107 of the slit 108 and an aperture 106 which has a smaller diameter than the first filter 104 and accommodates the first filter 104. Further, in a cover 109 formed are an air aperture 111 and an aperture 110 which has a smaller diameter than the first filter 104 and accommodates the first filter 104.

Herein, while the filter holding plate 12 in Embodiment 1 above is comprised of one tabular body, a filter holding plate 112 in Embodiment 2 is comprised of filter holding plates 112a, 112b and 112c.

In the filter holding plate 112a formed is a space 113a with a diameter larger than the diameter of the first filter 104; in the filter holding plate 112b formed are a space 113b with the same diameter as the diameter of the space 113a, and a protruding filter holding part 114 (protruding part) which directly supports the first filter 104. The filter holding plate 112c is of the same shape as the filter holding plate 112a and a space 113c is formed therein.

Moreover, an opening 116 communicating to the upper surface of the first filter 104 is formed in an upper cover 115 constituting the sample solution dropping part.

In integration of the aforesaid base plate 101, spacer 105, cover 109, filter holding plates 112a, 112b and 112c, and upper cover 115, the aperture 106 leading to the slit 108, the aperture 110 formed in the cover 109, the space 113a formed in the filter holding plate 112a, the space 113b formed in the filter holding plate 112b, the space 113c formed in the filter holding plate 112c, and the opening 116 formed in the upper cover 115 are communicated.

The first filter 104 as a hemocyte-filtering part is made of glass fiber filter paper and has the shape of a cylinder with a pore size of 0.5 to 5 *µ*m, a diameter of 3 mm and a height (thickness) of 500 to 1,000 *µ*m. Fibers constituting the glass fiber filter paper are coated with polyvinyl alcohol (PVA).

For assembly of this sensor, first, the cover 109 is placed on the spacer 105 based on the positional relationship shown in FIG. 5 to obtain a combined base plate C. As later descried, a reaction layer is formed in a concave portion (sample solution supply pathway) formed by the slit 108 when the cover 109 is combined with the space 105. Further, based on the positional relationship shown in FIG. 5, the filter holding plate 112b and then the filter holding plate 112c are placed on the filter holding plate 112a, and the upper cover 115 is further placed thereon to obtain a combined base plate D.

The base plate 101 is combined with the combined base plate A based on the positional relationship shown in FIG. 5 and the first filter 104 is disposed directly on the communicating apertures 106 and 110. Subsequently, the base plate 101, the combined base plate C and the combined base plate D are combined in such a positional relationship that the right ends of these members are aligned.

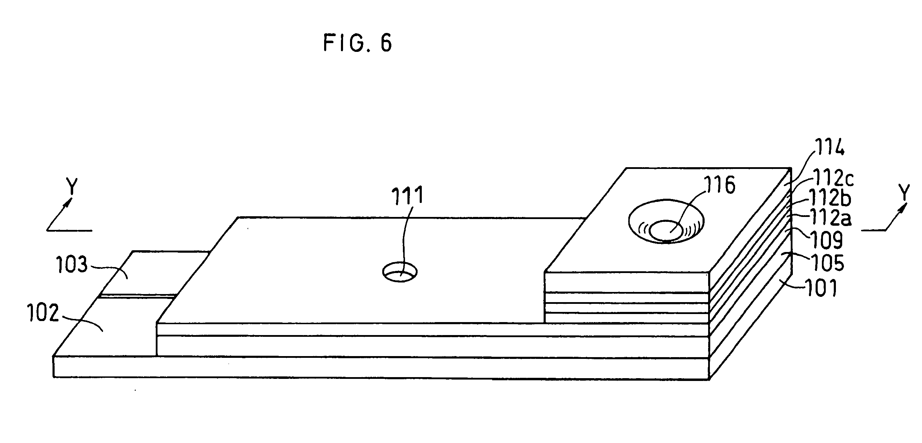

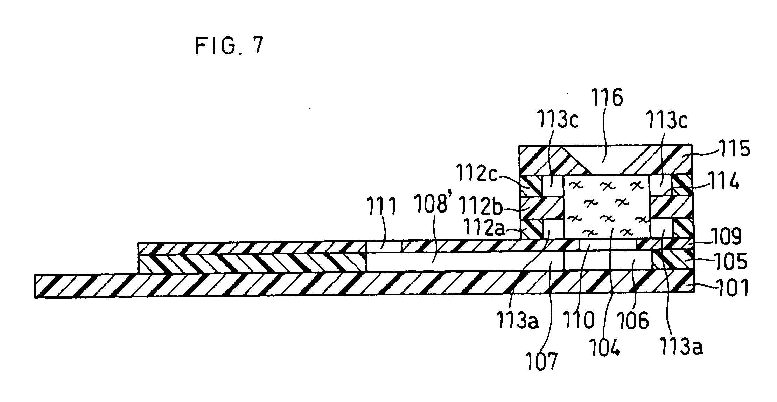

Herein, a schematic perspective view of a biosensor in accordance with the present invention obtained according to the configuration of FIG. 5 is shown in FIG. 6. A sectional view taken on the line Y-Y of FIG. 6 is further shown in FIG. 7.

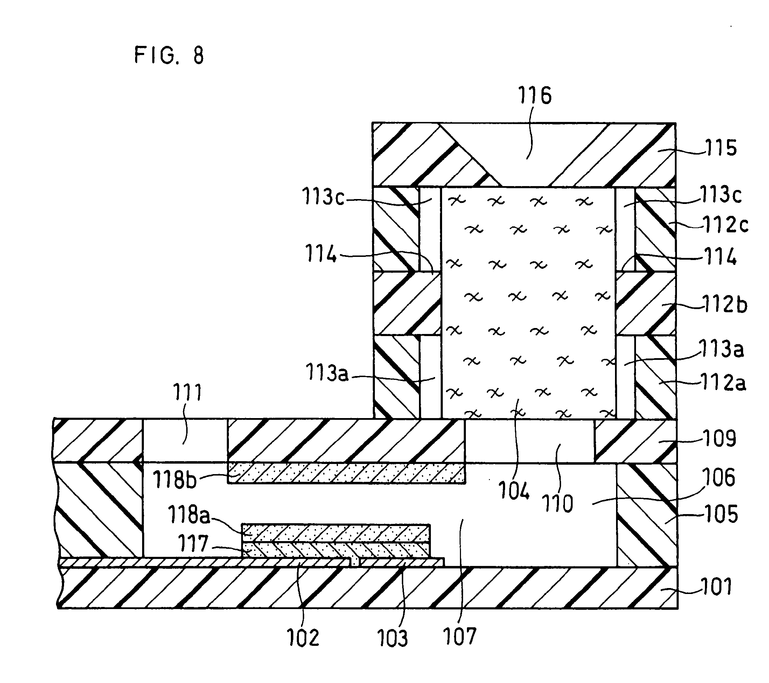

The reaction layer and the electrode system are omitted from FIG. 7 whereas a partially enlarged sectional view corresponding to FIG. 7 which represents the reaction layer and the electrode system is shown in FIG. 8. On the electrode system (102 and 103) of the base plate 101 formed are a hydrophilic polymer layer 117 and a reaction layer 118a. Further, a reaction layer 118b is formed on the lower face of the cover 109 corresponding to the ceiling of the sample solution supply pathway. It should be noted that the other members shown in FIG. 8 are equivalent to those shown in FIG. 7.

The biosensors of the present invention shown in FIGS. 5 to 8 are produced using the first filter 104 and seven types of base plates so as to make the structures thereof easy to understand. However, the cover 109 and the spacer 105 can be composed of one member. Further, the filter holding plates 112a and 112b, or the filter holding plates 112b and 112c, may be omitted, depending on the thickness of the first filter 104. Moreover, the filter holding part 114 may be omitted.

For measurement of cholesterol in blood with the use of the biosensor shown in FIGS. 1 to 4 or FIGS. 5 to 8, for example, blood as the sample solution taken from the fingertip by puncturing is supplied to the opening 16 or 116 of the upper cover 15 or 115. The blood supplied here infiltrates from the sample solution supply part side into the first filter to be filtered therein. Plasma as a filtrate other than the hemocytes exudes from the sample solution supply pathway side of the filter. The plasma having exuded fills the entire sample solution supply pathway 8' or 108' constituted by the slit 8 or 108 extended to the vicinity of the electrode system and further to the air aperture 11 or 111, while dissolving a reaction layer carried on the position covering the electrode system and/or the reverse face of the cover 9 or 109. Once the entire sample solution supply pathway 8' or 108' is filled, the flow of the liquid dropped onto the filter 4 or 104 also stops.

As the first filter 4 or 104 preferably used is one having a discontinuous, heterogeneous internal structure as well as a nonuniform pore size distribution. It is therefore preferable that the form of the flow channel inside the first filter 4 or 104 is also irregular. It should be noted that the filtration is performed inside the filter 4 or 104. Further, in Embodiment 2 above, fibers constituting the filter 104 are made coated with PVA.

After undergoing such a hemocyte-filtering process, a chemical reaction of the reaction layer dissolved by the plasma with a component to be measured (e.g. glucose, total cholesterol, low-density lipoprotein (LDL) cholesterol, etc.) in the plasma occurs, and a current value in the electrode reaction is measured after a lapse of a certain period of time to determine the component in the plasma.

As thus described, FIGS. 4 and 8 show examples of disposition of the reaction layer in the vicinity of the electrode system in the sample solution supply pathway 8' or 108'. On the electrode systems of 2 and 3 or 102 and 103 of the base plate 1 or 101 formed are the hydrophilic polymer layer 17 or 117 including carboxymethyl cellulose (hereinafter simply referred to as "CMC") or the like, as well as the reaction layer 18a or 118a including a reaction reagent such as the electron mediator. The reaction layer 18b or 118b including oxidoreductase is formed on the surface, exposed to the sample solution supply pathway 8' or 108', of the cover 9 or 109 on the combined base plate A or C obtained by combining the cover 9 or 109 with the spacer 5 or 105.

In Embodiment 1 above, in particular, the aperture 10 in the filter holding plate 12 is designed in such a manner that it is not in contact with the first filter 4, except the filter holding part 14, to avoid interfering expansion of the first filter 4. This can eliminate the fear of destructing the hemocytes in the sample solution.

In the biosensor having the structures shown in FIGS. 1 to 4, it is preferable that the sample solution supply pathway has a width of not more than 1.5 mm, a height of not more than 150 *µ*m, and a length of not more than 4.5 mm.

The volume of the biosensor in accordance with the present invention is preferably not less than 0.05 *µ*l and not more than 1.0125 *µ*l. It is further preferable that the electrode system is constituted by the electrodes comprising noble metal. With the preferable width of the sample solution supply pathway being not more than 1.5 mm, an electrode area of a printing electrode obtained by means of screen-printing is determined with poor accuracy. In regard to the noble metal electrode, however, a laser trimming can be performed by a 0.1 mm width and the electrode area is thus determined with high accuracy.

It is to be noted that, especially in the biosensor in accordance with Embodiment 1, at least part of the sample solution supply pathway inlet side of the first filter 4 may be in contact with the base plate 1, the spacer 5 and the cover 9, as shown in FIG. 13, e.g. the sample solution supply pathway inlet side of the first filter 4 may be cut crosswise to give a tilt part, as shown in FIG. 14. As thus described, the provision of the tilt part in such a shape as getting wider toward the inlet 7 on the face in contact with the base plate 1 makes it easier to introduce the filtrate into the sample solution supply pathway 8'.

As shown in FIG. 13, it is preferable that in the first filter, the cross sectional area (F1) of the sample solution supply part side is larger than the cross sectional area (S1) of the inlet 7 of the sample solution supply pathway. This can produce an effect that the inside of the sample solution supply pathway 8' in the sensor is saturated with the filtered plasma at a faster rate.

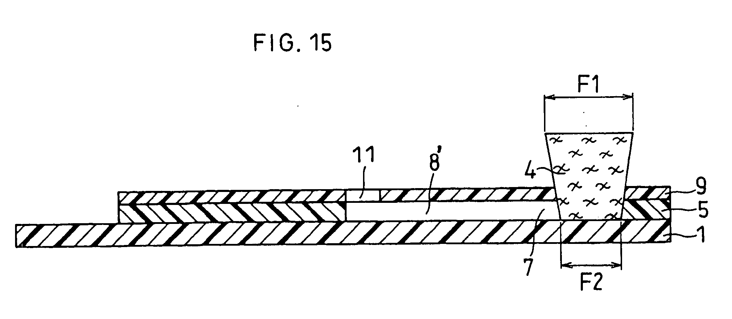

As shown in FIG. 15, it is further preferable that in the first filter, the cross sectional area (F1) of the sample solution supply part side is larger than the cross sectional area (F2) of the sample solution supply pathway inlet side in contact with the base plate 1 on the side of sample solution supply pathway inlet 7. This can produce an effect that the inside of the sample solution supply pathway 8' in the sensor is saturated with the filtered plasma at still a faster rate. It is to be noted that constituents other than the base plate 1, the first filter 4, the spacer 5 and the cover 9 were omitted from the FIGS. 13 to 15.

### Embodiment 3

A biosensor in accordance with Embodiment 3 of the present invention comprises: an insulating base plate; an electrode system having a measuring electrode and a counter electrode which are provided on the base plate; a reaction layer including at least oxidoreductase and an electron mediator; a sample solution supply pathway which includes the reaction layer in contact with the base plate and has an air aperture at the end; a sample solution dropping part for introducing a sample solution; and a first filter which is disposed between the sample solution supply pathway and the sample solution dropping part, without intruding into the sample solution supply pathway, and filters the sample solution in the vertical direction, where a filtrate is absorbed into the sample solution supply pathway due to capillary action and passes laterally from the inlet of the sample solution supply pathway toward the air aperture thereof, characterized in that the first filter is constituted by a membrane filter with a uniform pore size free of deformation and the lower part of the first filter is not in contact with the electrode system.

The examples of materials for constituting the membrane filter may include polyester polycarbonate and nitrocellulose. The use of such a membrane filter allows simplification of a structure of an obtained biosensor, prevention of a measurement error caused by mixing of hemocytes and, further, reduction in sample amount.

Moreover, in the biosensor of the present invention, the vertical separation method is employed as the method for separating hemocytes. Since the first filter is constituted by the membrane filter having a continuous, homogenous internal structure, a uniform pore size and a regular internal flow channel, almost a 100% capture rate is exerted. It is preferable in this case that the pore size is from 0.1 to 10 *µ*m and the thickness is from 5 to 30 *µ*m.

The use of glass fiber filter paper as the first filter requires a large amount of the sample solution since the sample solution is filtered within the filter and absorbed in the filter itself in a considerable amount. With the use of the membrane filter, on the other hand, the sample solution is filtered through the filter surface and it will therefore not be absorbed in the filter itself so that the amount of the sample solution to be used can be reduced.

It is also preferable in the biosensor in accordance with the present invention that a hydrophilic processing part is provided between the first filter and the base plate.

When whole blood is used as the sample solution, plasma which has eluded from the sample solution supply pathway inlet side of the first filter by the filtration develops onto the base plate via this hydrophilic layer due to capillary action, and further moves toward where the electrode system is provided.

The plasma then intrudes into the sample solution supply pathway from the inlet of the sample solution supply pathway to fill the entire sample solution supply pathway to the air aperture.

Further, since the first filter does not intrude into the sample solution supply pathway, as in the structure of the biosensor described in Japanese Patent Application No. 2001-180362, the filter is not in contact with the electrode system while being in contact with the base plate. It is thereby possible to realize stable sensor accuracy without occurrence of a measurement error attributed to the contact between the filter and the electrode system.

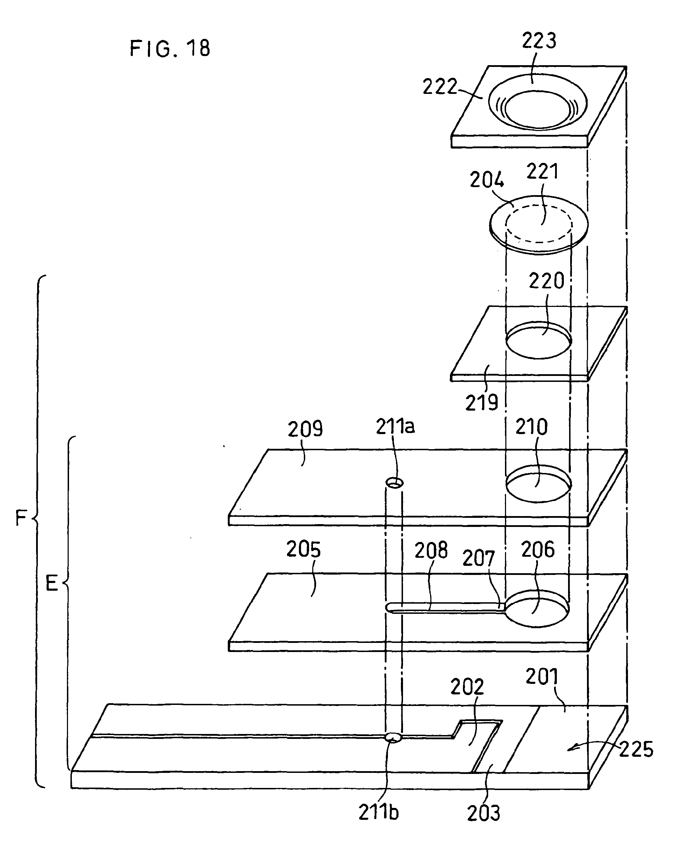

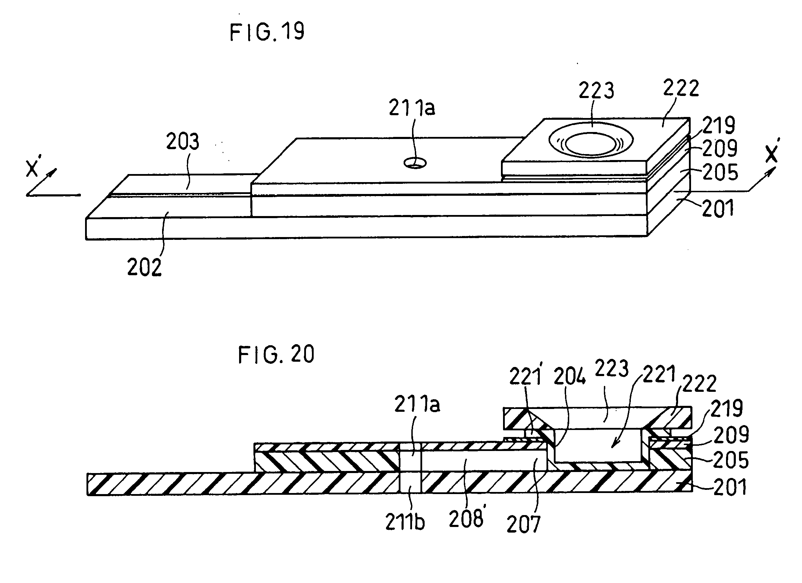

FIG. 18 is an exploded perspective view of the biosensor in accordance with the preferable embodiment of the present invention. Further, FIG. 19 is a combined perspective view of the biosensor shown in FIG. 18, and FIG. 20 is a vertical sectional view of the biosensor taken on the line X'-X' shown in FIG. 19 with the reaction layer and the like omitted therefrom.

As shown in FIG. 18, the biosensor in accordance with the present invention is constituted by an insulating base plate 201, a spacer 205, a cover 209, a double-faced tape 219, a first filter 204 and a sample solution dropping part 222.

The insulating base plate 201 is made of an insulating resin such as polyethylene terephthalate. On the left upper face of the insulating base plate 201, a palladium portion is formed by means of sputtering, vapor deposition or the like, followed by laser trimming, to form an electrode system including a working electrode 202 and a counter electrode 203.

Moreover, an air aperture 211b is formed in the insulating base plate 201, and the area of the electrode system is determined by a width of a slit 208 formed on the spacer 205, as later described. Further, the right end 225 of the insulating base plate 201 in contact with the first filter 204 is processed to become hydrophilic, as subsequently described.

In the spacer 205 to be combined with the insulating base plate 201 formed are the slit 208 for forming the sample solution supply pathway, an inlet 207 of the slit 208 and an aperture 206 for accommodating the first filter 204, in the biosensor obtained after being assembled.

Further, in a cover 209 formed are an air aperture 211a and an aperture 210 for accommodating the first filter 204, and when the cover 209, the spacer 205 and the insulating base plate 201 are combined, the air aperture 211a communicates to an air aperture 211b formed in the insulating base plate 201 via the sample solution supply pathway.

An aperture 220 for accommodating the first filter 204 is formed in the double-faced tape 219 disposed on the upper face of the cover 209. The double-faced tape used here may be one capable of bonding the sample solution dropping part 222 to the cover 209, while holding the first filter 204 between the tape and the sample solution dropping part 222. Hence a tabular body having a bonding layer on each face thereof may be used other than the double-faced tape.

Furthermore, an aperture 223 communicating to the first filter 204 is formed in the sample solution dropping part 222.

In integration of the aforesaid insulating base plate 201, spacer 205, cover 209, and double-faced tape 219, the aperture 206 leading to the slit 208, the aperture 210 formed in the cover 209 and the aperture 220 formed in the double-faced tape 219 are communicated. Further, the air aperture 211b formed in the insulating base plate 201 and the air aperture 211a formed in the cover 209, as thus described, are communicated.

The first filter 204 for separating hemocytes is constituted by the membrane filter and may have such a degree of pore size that the hemocytes cannot pass therethrough (e.g. 5 *µ*m).

Further, although the first filter 204 is in the shape of a circle with a diameter of 5 mm, for example, before assembly of the biosensor in accordance with the present invention, a pit 221 is formed just before the assembly and, in a biosensor after being assembled, the first filter 204 is disposed in such a manner as being in substantially cylindrical shape having an opening in the upper part thereof and a circular circumferential part, as shown in FIG. 20.

For assembly of this biosensor, first, a reaction layer 218a is formed on a prescribed portion according to the need of the insulating base plate 201, as subsequently described. Further, as shown in FIG. 18, the insulating base plate 201, the cover 209 and the spacer 205 are combined such that the right ends of these members are aligned, to obtain a combined base plate E; as later described, a reaction layer 218b is formed on a prescribed member in a sample solution supply pathway 208' (see FIG. 20) which is a concave portion formed by the slit 208.

Subsequently, the combined base plate E is combined with the double-faced tape 219 such that the right ends of these members are aligned, to produce a combined base plate F, and the first filter 204 is disposed directly above and on the communicating apertures 206, 210 and 220.

When the first filter 204 and the double-faced tape part 219 are mutually bonded, for example, the central portion of the first filter 204 is lightly pressed in advance with the use of a cylindrical stick made of foam polystyrene or the like, which is resistant to scratching the filter, to form the pit 221. A circumferential part 221' (see FIG. 20) off the aperture formed by the pressing with the stick is bonded to the double-faced tape 219 and the stick is taken out after the entire circumferential part 221' has been bonded to the double-faced tape 219.

Finally, the sample solution dropping part 222 is disposed. The aperture 223 in the sample solution dropping part 222 communicates to the pit 221 in the first filter 204.

As shown in the sectional view in FIG. 20, in the case of using whole blood as the sample solution in the biosensor in accordance with the present invention, complete removal of hemocytes as interfering substances requires the sample solution to certainly pass through the filter 204. Further, rapid absorption of the filtered plasma and rapid supply thereof into the sample solution supply pathway 208' require the first filter 204 to be disposed in the vicinity of the inlet 207 of the sample solution supply pathway 208' without intruding into the sample solution supply pathway 208'.

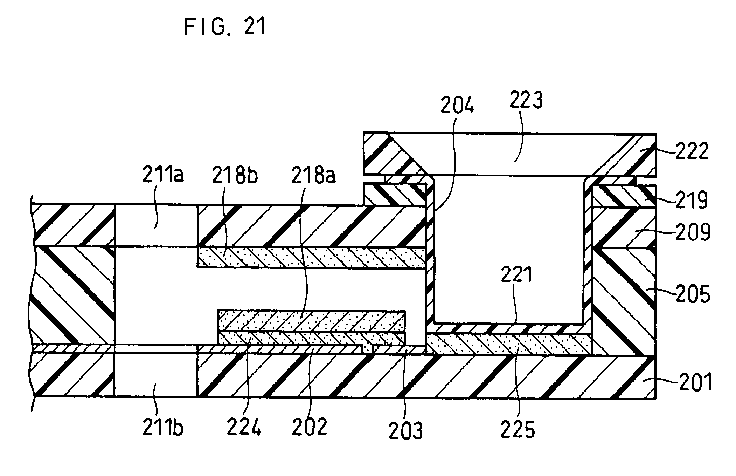

The reaction layer and the electrode system are omitted from FIG. 19 whereas a partially enlarged view corresponding to FIG. 20 which represents the reaction layer and the electrode system is shown in FIG. 21. On the electrodes 202 and 203 of the base plate 201 formed are a hydrophilic polymer layer 224 and a reaction layer 218a. Further, a reaction layer 218b is formed on the lower face of the cover 209 corresponding to the ceiling of the sample solution supply pathway.

It is to be noted that, although the biosensor shown in FIGS. 18 to 21 is produced using five types of members so as to make the structures thereof easy to understand, the combined base plate E obtained by combining the spacer 205 with the cover 209 may be composed of a single member.

Next, for measurement of a substrate (e.g. cholesterol) in blood with the use of the biosensor shown in FIGS. 18 to 21, blood as the sample solution is added dropwise onto the aperture 223 of the sample solution dropping part 222. Out of the dropped blood, only plasma is captured on the upper surface of the sample solution supply part side of the first filer 204 and only the plasma exudes from the surface of the sample solution supply pathway inlet side. The plasma having exuded intrudes into the sample solution supply pathway 208' through the inlet 207 and fills the sample solution supply pathway 208' while dissolving a reaction layer carried on the position covering the electrode system and/or the reverse face of the cover 209.

More specifically, the plasma having exuded from the first filter 204 fills the entire sample solution supply pathway 208' extended to the vicinity of the electrode and further to the portion of the air apertures 211b and 211a. Once the entire sample solution supply pathway 208' is filled with the liquid, the flow of the liquid in the filter 204 also stops.

After undergoing such a hemocyte-filtering process, a chemical reaction of the reaction layer dissolved by the plasma with a component to be measured (e.g. cholesterol) in the plasma occurs, and a current value in the electrode reaction is measured after a lapse of a certain period of time to determine the component in the plasma.

Herein, FIG. 21 is a vertical sectional view showing an example of disposition of the reaction layer in the vicinity of the electrode system in the sample solution supply pathway 208' and the hydrophilic layer provided on the interface between the first filter 204 and the insulting base plate 201.

On the electrode system of the insulating base plate 201 formed are a hydrophilic layer 224 including a hydrophilic polymer such as CMC and the reaction layer 218a including a reaction reagent such as the electron mediator.

Further, the reaction layer 218b including oxidoreductase is formed on the face, exposed to the sample solution supply pathway 208', of the cover 209 on the combined base plate E obtained by combining the insulating base plate 201, the cover 209 and the spacer 205. A hydrophilic layer 225 including a hydrophilic polymer such as CMC is formed at the right end of the insulating base plate 201, namely the portion where the insulating base place 201 is in contact with the first filter 204.

It is preferable that the electrode system comprises noble metal electrodes. With the preferable width of the sample solution supply pathway 208' being not more than 2 mm, an electrode area of a printing electrode obtained by means of screen-printing is determined with poor accuracy. When the noble electrodes are used, on the contrary, a laser trimming can be performed by a 0.1 mm width and the electrode area is thus determined with high accuracy.

### Embodiment 4

A biosensor in accordance with Embodiment 4 of the present invention comprises: an insulating base plate; an electrode system having a measuring electrode and a counter electrode which are provided on the base plate; a reaction layer including at least oxidoreductase and an electron mediator; a sample solution supply pathway including the base plate and the reaction layer; an air aperture provided on the end side of the sample solution supply pathway; a sample solution dropping part for introducing the sample solution, a first filter which is disposed between the sample solution supply pathway and the sample solution dropping part, without intruding into the sample solution supply pathway, and filters the sample solution in the vertical direction; and a second filter located on the downstream side of the first filter, where a filtrate is absorbed into the sample solution supply pathway due to capillary action and passes laterally from the inlet of the sample solution supply pathway toward the air aperture thereof, characterized in that the mean pore size of the first filter is smaller than the mean pore size of the second filter.

As thus described, in the conventional technique using two types or more of blood cell separators, since an uppermost filter is used for pretreatment and an undermost filter is used for terminal treatment, the pore size of the uppermost filter has always been larger than that of the undermost filter (US Patent No. 5,240,862, and WO publication No. 96/15453).

As opposed to this, in the biosensor of the present invention using two types of filters, i.e. a first filter and a second filter, the first filter serves to separate hemocytes almost completely and hence a very small amount of the hemocytes arrive at the second filter. The second filter serves to absorb the very small amount of the filtrate getting out of the sample solution supply pathway inlet side and to supply this filtrate into the sample solution supply pathway.

Although there is a tendency that direct supply of the filtrate from the first filter with a small pore size to the hollowing sample solution supply pathway is impossible unless pressure is applied, the provision of the second filter between the first filter and the sample solution supply pathway allows complete filtration of the hemocytes as well as rapid supply of only a filtrate having passed through the second filter into the sample solution supply pathway. It further allows complete prevention of the hemocytes from flowing into the sample solution supply pathway so that the problem of a measurement error can be eliminated.

Moreover, for rapid absorption of the filtrate from the second filter into the sample solution supply pathway due to capillary action, the volume of the sample solution supply pathway has importance and in the present invention, it is preferably not more than 2 *µ*l. Capillary action generally refers to a phenomenon that, when a capillary is put up in a liquid, the liquid level in the capillary becomes higher (or lower) than the liquid level outside the capillary. An amount of the liquid to be sucked into the capillary is determined by the strength of the interaction (adhesion) between the surface tension of the liquid and the capillary wall, and it is therefore possible to make a greater amount of the liquid sucked into a capillary in the case of employing a thin capillary than the case of employing a thick capillary. Accordingly, the sample solution supply pathway preferably has the structure equivalent to a thin capillary with a volume of not more than 2 *µ*l in order to rapidly supply the filtrate into the sample solution supply pathway.

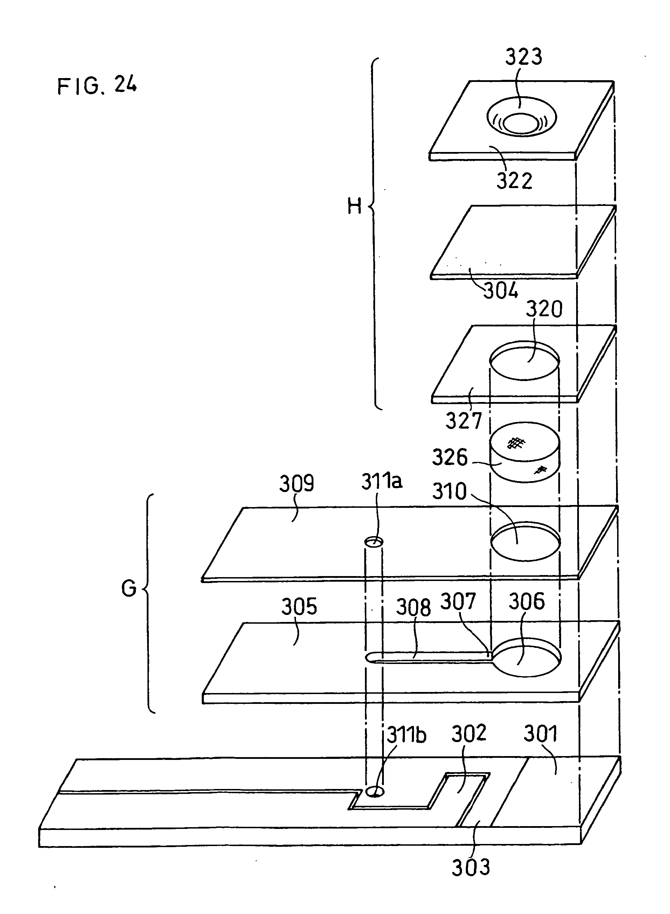

FIG. 24 is an exploded perspective view of the biosensor in accordance with the preferable embodiment of the present invention. The biosensor in accordance with the present invention shown in FIG. 24 has an insulating base plate 301 made of an insulating resin such as polyethylene terephthalate. In FIG. 24, a palladium portion is formed on the left upper face of the insulating base plate by means of sputtering, vapor deposition or the like, followed by laser trimming, to form an electrode system including a working electrode 302 and a counter electrode 303. The electrode area may be determined by a width of a slit 308 formed on a spacer 305, as later described.

In the spacer 305 to be combined with the insulating base plate 301 formed are the slit 308 for forming the sample solution supply pathway, an inlet 307 of the slit (sample solution supply pathway) and an aperture 306 for accommodating a second filter 326, in the biosensor obtained after being assembled. An air aperture 311a and an aperture 310 for accommodating the second filter 326 are formed in a cover 309, an aperture 320 is formed in a spacer 327, and an aperture 323 communicating to the filter 304 is formed in a sample solution dropping part 322.

In integration of the aforesaid base plate 301, spacer 305, cover 309, and spacer 327, the aperture 306 leading to the slit 308, the aperture 310 formed in the cover 309 and the aperture 320 formed in the spacer 327 are communicated.

The first filter 304 for separating hemocytes is constituted by a membrane filter and the size of the pore (pore size) thereof may be in such a degree that hemocytes cannot pass therethrough (e.g. 0.1 to 5 *µ*m). For example, the first filter 304 is cut out to be a 6 mm square. The thickness may be on the order of 0.1 to 40 *µ*m.

The second filter 326 for absorbing plasma is constituted by glass fiber filter paper and has been cut out to be in the shape of a cylinder with a diameter of 2.5 mm and a height (thickness) of 500 to 1,000 *µ*m. It is preferable that the pore size thereof is not less than 0.1 *µ*m and is larger than the pore size of the first filter.

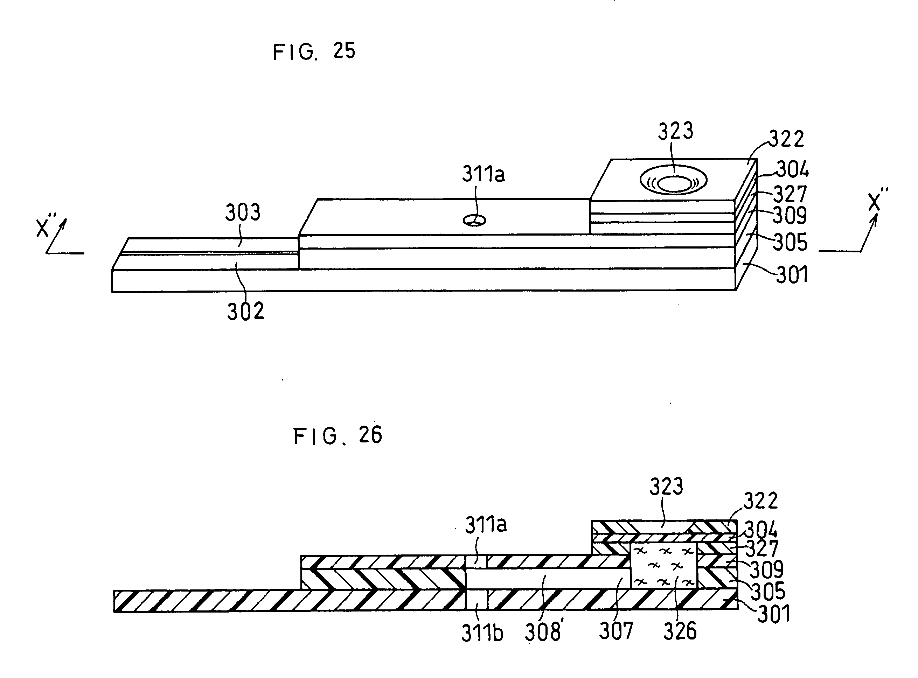

For assembly of this biosensor, in a sample solution supply pathway 308' (see FIG. 26) in concave form formed by the slit 308 when the cover 309 is combined with the spacer 305, a reaction layer is formed on a prescribed member, as later described, and the insulating base plate 301, the spacer 305, the cover 309 and the spacer 327 are then combined in such a manner that the right ends of these members are aligned.

Subsequently, as shown by the dotted lines in FIG. 24, the second filter 326 is disposed in the communicating aperture formed by the apertures 306, 310 and 320. Finally, the first filter 304 is disposed on the spacer 327, on which the sample solution dropping part 322 is further disposed. The aperture 323 of the sample solution dropping part 322 is communicated to the first filter 304.

A schematic perspective view of the biosensor in accordance with present invention obtained according to the configuration of the FIG. 24 is shown in FIG. 25. A sectional view taken on the line X''-X'' of FIG. 25 is further shown in FIG. 26. It should be noted that the reaction layer, the electrode system and the like are omitted from FIG. 26.

As shown in the sectional view in FIG. 26, complete removal of hemocytes as interfering substances from whole blood as the sample solution in the biosensor in accordance with the present invention requires the sample solution to certainly pass through the first filter 304. Rapid absorption of the filtered plasma and rapid supply thereof into the sample solution supply pathway 308' require the second filter 326 to be in contact with the first filter 304 as well as the inlet 307 of the sample solution supply pathway 308'.

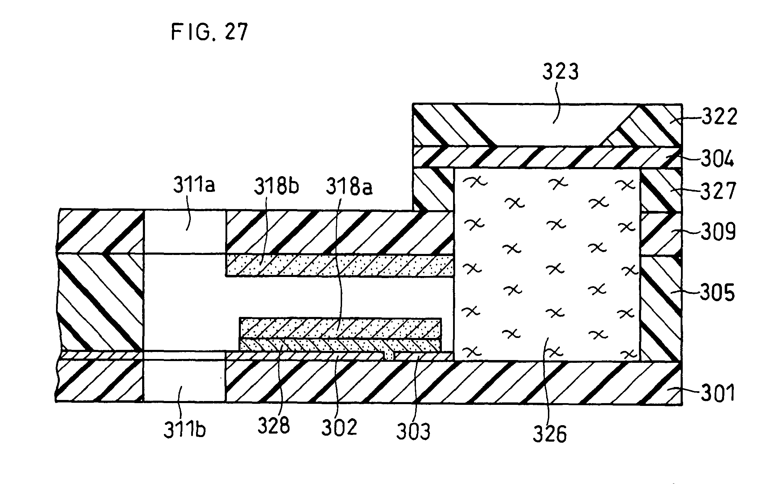

The reaction layer and the electrode system are omitted from FIG. 26 whereas a partially sectional view corresponding to FIG. 26 which represents the reaction layer and the electrode system is shown in FIG. 27. On the electrodes 302 and 303 of the insulating base plate 301 formed are a hydrophilic polymer layer 328 and a reaction layer 318a. Further, a reaction layer 318b is formed on the lower face of the cover 309 corresponding to the ceiling of the sample solution supply pathway 308'.

The biosensor shown in FIGS. 24 to 27 is produced using five types of members so as to make the structures thereof easy to understand. However, the spacer 305 and the cover 309 may be formed by one member G, which may be added with the spacers 327, 304 and 322 to be formed altogether by one member H. Or the spacer 327 may be removed, depending on the thickness of the second filter 326.

For measurement of cholesterol in blood with the use of the biosensor shown in FIGS. 24 to 27, blood as the sample solution is added dropwise onto the aperture 323 of the sample solution dropping part 322. With whole blood dropped here, only plasma is captured on the upper surface of the sample solution supply part side of the first filer 304 and only the plasma exudes from the surface of the sample solution supply pathway side of the first filter. The plasma having exuded fills the entire second filter 326. When the inside of the second filter becomes saturated with the plasma, the plasma intrudes into the sample solution supply pathway 308' through the inlet 307 and fills the inside of the sample solution supply pathway 308', while dissolving a reaction layer carried on the position covering the electrode system and/or the reverse face of the cover 309. More specifically, the plasma having exuded from the second filter 326 fills the entire sample solution supply pathway 308' extended to the vicinity of the electrode and further to the portion of the air aperture 311a. Once the entire sample solution supply pathway 308' is filled with the liquids, the flow of the liquids in the second filter 326 and the first filter 304 also stop.

After undergoing such a hemocyte-filtering process, a chemical reaction of the reaction layer dissolved by the plasma with a component to be measured (e.g. cholesterol in the case of a cholesterol sensor) in the plasma occurs, and a current value in the electrode reaction is measured after a lapse of a certain period of time to determine the component in the plasma.

As thus described, FIG. 27 shows an example of disposition of the reaction layer in the vicinity of the electrode system in the sample solution supply pathway 308'. On the electrode system of the base plate 301 formed are a layer 328 of a hydrophilic polymer such as CMC and the reaction layer 318a including a reaction reagent such as the electron mediator. Further, the reaction layer 318b including oxidoreductase is formed on the face, exposed to the sample solution supply pathway, within the reverse face of a cover member obtained by combining the cover 309 with the spacer 305.

It is preferable that the electrode system comprises noble metal electrodes. With the preferable width of the sample solution supply pathway 308' being not more than 2 mm, an electrode area of a printing electrode obtained by means of screen-printing is determined with poor accuracy. When the noble electrodes are used, on the contrary, a laser trimming can be performed by a 0.1 mm width and the electrode area is thus determined with high accuracy.

Below, examples of the present invention are described; however, the present invention is not limited thereto.

### Example 1

A biosensor was produced which had the structures in accordance with Embodiment 1 shown in FIGS. 1 to 4, whose object to be measured was total cholesterol and in which the reaction layer 18a included the electron mediator and the reaction layer 18b included cholesterol oxidase, cholesterol esterase and a surfactant.

First, 5 *µ*l of an aqueous solution containing 0.5 wt% of CMC was dropped onto the electrode system of the base plate 1, and dried in a drying apparatus with warm blast at 50°C for 10 minutes to form the hydrophilic polymer layer 17. Next, 4 *µ*l of a potassium ferricyanide aqueous solution (corresponding to 70 mM of potassium ferricyanide) was dropped onto the hydrophilic polymer layer 17, and dried in the drying apparatus with warm blast at 50°C for 10 minutes to form the reaction layer 18a including potassium ferricyanide.

Polyoxyethylene (10) octyl phenyl ether shown as TritonX-100 as the surfactant was added to an aqueous solution with cholesterol oxidase (EC1.1.3.6 : ChOD) originating from Nocardia and cholesterol esterase (EC.3.1.1.13 : ChE) originating from Pseudomonas dissolved therein. 0.4 *µ*l of this mixed solution was dropped onto the concave part (a part corresponding to the upper side of the sample solution supply pathway 8' of the cover 9) formed by integrating the cover 9 with the spacer 5, prefrozen with liquid nitrogen at -196°C, and dried in a freeze-drying apparatus for two hours, to form the reaction layer 18b (FIG. 4) including 450 U/ml of cholesterol oxidase, 1,125 U/ml of cholesterol esterase and 2 wt% of the surfactant.

It is to be noted that when a biosensor is obtained whose object to be measured is LDL cholesterol, a surfactant capable of selectively solubilizing LDL may be used. This introduces LDL cholesterol to the reaction system to allow the measurement thereof. Since enzyme reactions to other lipoprotein than LDL (chylomicron, high density lipoprotein (HDL), very low density lipoprotein (VLDL)) are inhibited by this surfactant, these lipoproteins are not introduced to the reaction system of cholesterol and remain in the reaction solution in the form of lipoproteins.

As for each size of the sensor, it is preferable that the slit width is 0.8 mm, the slit length (the length between the inlet and the air aperture of the sample solution supply pathway) is 4.5 mm, the thickness of the spacer 5 (the distance between the base plate 1 and the cover 9) is 100 *µ*m.

The first filter 4 was produced by punching out glass fiber filter paper with a thickness of about 700 *µ*m in the shape of a circle with a diameter of 2.5 mm to be in cylindrical shape. The base plate 1, the spacer 5, the cover 9 and the filter holding plate 12 were combined to give a space, comprising the aperture 6, the aperture 10 and the space 13, which was provided with the first filter 4.

Subsequently, the upper cover 14 was disposed on the upper part of the filter holding plate 12 to produce a biosensor having the structures shown in FIGS. 1 to 4.

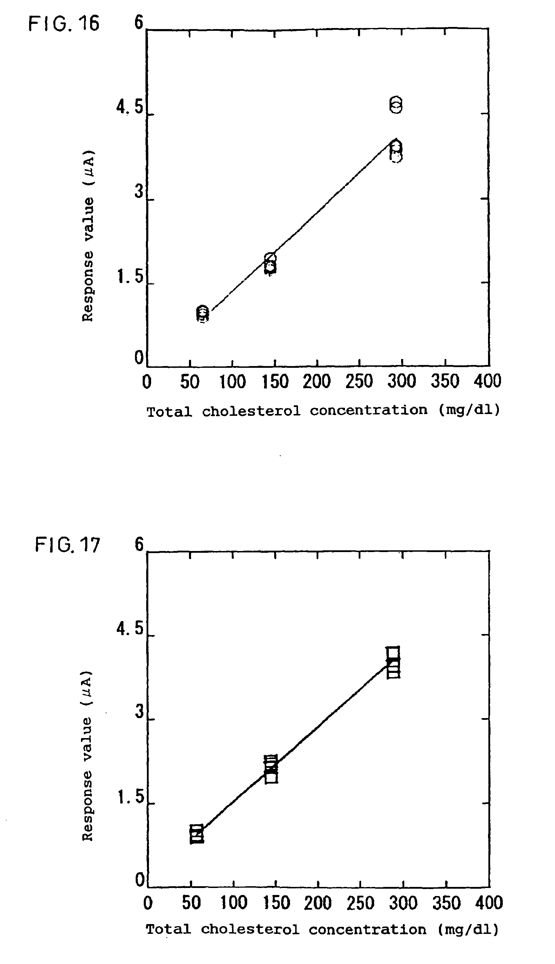

10 *µ*l of whole blood or a standard serum as the sample solution was dropped from the opening 16 of the obtained biosensor, 180 seconds layer, a pulse voltage of +0.2 V was applied to the working electrode toward the anode relative to the counter electrode, and five seconds later, a value of a current flowing between the working electrode and the counter electrode was measured. The resultant response characteristic was shown in FIG. 16.

As is evident from FIG. 16, according to the biosensor in accordance with the present invention, a favorable linearity between the total cholesterol concentration and the response current value can be obtained.

### Example 2

A biosensor was produced which had the structures in accordance with Embodiment 2 shown in FIGS. 5 to 8, whose object to be measured was total cholesterol and in which the reaction layer 118a included the electron mediator and the reaction layer 118b included cholesterol oxidase, cholesterol esterase and a surfactant.

First, 5 *µ*l of an aqueous solution containing 0.5 wt% of CMC was dropped onto the electrode system of the base plate 101, and dried in a drying apparatus with warm blast at 50°C for 10 minutes to form the hydrophilic polymer layer 117. Next, 4 *µ*l of a potassium ferricyanide aqueous solution (corresponding to 70 mM of potassium ferricyanide) was dropped onto the hydrophilic polymer layer 117, and dried in the drying apparatus with warm blast at 50°C for 10 minutes to form the reaction layer 118a including potassium ferricyanide.

Polyoxyethylene (10) octyl phenyl ether shown as TritonX-100 as the surfactant was added to an aqueous solution with cholesterol oxidase (EC1.1.3.6 : ChOD) originating from Nocardia and cholesterol esterase (EC.3.1.1.13 : ChE) originating from Pseudomonas dissolved therein. 0.4 *µ*l of this mixed solution was dropped onto the concave part (a part corresponding to the upper side of the sample solution supply pathway 108' of the cover 109) formed by integrating the cover 109 with the spacer 105, prefrozen with liquid nitrogen at -196°C, and dried in a freeze-drying apparatus for two hours, to form the reaction layer 118b (FIG. 8) including 450 U/ml of cholesterol oxidase, 1,125 U/ml of cholesterol esterase and 2 wt% of the surfactant.

It is to be noted that when a biosensor is obtained whose object to be measured is LDL cholesterol, a surfactant capable of selectively solubilizing only LDL may be used. This introduces LDL cholesterol to the reaction system to allow the measurement thereof. Since enzyme reactions to other lipoprotein than LDL (chylomicron, HDL, VLDL) are inhibited by this surfactant, these lipoproteins are not introduced to the reaction system of cholesterol and remain in the reaction solution in the form of lipoproteins.

The first filter 104 was produced by punching out glass fiber filter paper coated with PVA, in the shape of a circle with a diameter of 2.5 mm.

Subsequently, the first filter 104 is formed on the combined base plate C which is on the base plate 101, followed by bonding of the combined base plate B obtained by integration of the filter holding plates 112a, 112b and 112c with the upper cover 115, onto the first filter 104, to produce a biosensor having the structures shown in FIGS. 5 to 8.

10 *µ*l of whole blood as the sample solution was dropped from the opening 116 of the obtained biosensor, 180 seconds layer, a pulse voltage of +0.2 V was applied to the measuring electrode toward the anode relative to the counter electrode, and five seconds later, a value of a current flowing between the working electrode and the counter electrode was measured. The resultant response characteristic was shown in FIG. 17.

As is apparent from FIG. 17, according to the biosensor in accordance with the present invention, a favorable linearity between the cholesterol concentration and the response current value can be obtained.

### Example 3

A biosensor was produced which had the structures in accordance with Embodiment 3 shown in FIGS. 18 to 21 and whose objects to be measured were total cholesterol and LDL cholesterol. The reaction layer 218a was added with the electron mediator and the reaction layer 218b was added with cholesterol oxidase, cholesterol esterase and a surfactant.

First, an aqueous solution containing 0.5 wt% of CMC was dropped onto the electrode system of the insulating base plate 201 as well as the face thereof in contact with the first filter 204, and dried in a drying apparatus with warm blast at 50°C for 10 minutes to form the CMC layer 224 and the hydrophilic layer 225.

Next, 4 *µ*l of a potassium ferricyanide aqueous solution (corresponding to 70 mM of potassium ferricyanide) was dropped onto the CMC layer 224, and dried in the drying apparatus with warm blast at 50°C for 10 minutes to form the reaction layer 218a including potassium ferricyanide.

Polyoxyethylene (10) octyl phenyl ether shown as TritonX-100 as the surfactant was added to an aqueous solution with cholesterol oxidase (EC1.1.3.6 : ChOD) originating from Nocardia and cholesterol esterase (EC.3.1.1.13 : ChE) originating from Pseudomonas dissolved therein. 0.4 *µ*l of the obtained mixed solution was dropped onto the portion, exposed to the sample solution supply pathway 208', of the cover 209, prefrozen with liquid nitrogen at -196°C, and dried in a freeze-drying apparatus for two hours, to form the reaction layer 218b (biosensor whose object to be measured was total cholesterol) including 450 U/ml of cholesterol oxidase, 1,125 U/ml of cholesterol esterase and 2 wt% of the surfactant (e.g. Emulgen B66 manufactured by Kao Corporation or a cation surfactant, with an HLB value of 13 to 15).

As the first filter 204 used here was one obtained by punching out, in the shape of a circle with a diameter of 5 mm, Cyclopore membrane (pore size 5.0 *µ*m, thickness 7.0 to 23 *µ*m) manufactured by Whatman Plc., Hema-fil membrane (pore size 5.0 *µ*m, thickness 11 *µ*m) manufactured by Corning Incorporated or Isopore membrane (pore size 5.0 *µ*m, thickness 10 *µ*m) manufactured by Millipore Corporation. This membrane was a continuously homogenous filter having a uniform pore size and a regular internal flow channel, and had a pore size of about 5 µm.

Thereafter, the combined base plate E and the combined base plate F were combined by alignment of the right ends of these members based on the positional relationship shown in FIG. 18, onto which the first filter 204 was further bonded to produce a biosensor having the structures shown in FIGS. 18 to 21.

### [Evaluation]

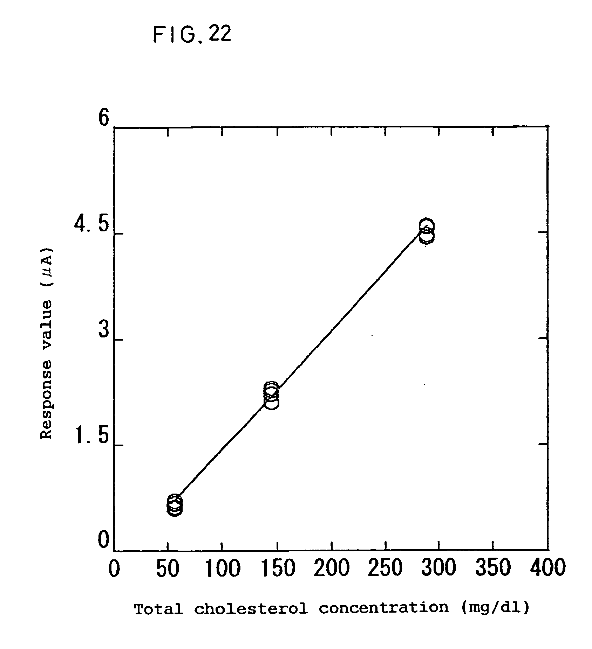

In the obtained biosensor, 5 µl of whole blood as the sample solution was added to the aperture 223 to serve as the sample solution adding part, 150 seconds layer, a pulse voltage of +0.2 V was applied to the measuring electrode toward the anode relative to the counter electrode, and five seconds later, a value of a current flowing between the working electrode and the counter electrode was measured. The results were shown in FIG. 22. FIG. 22 is a graph showing the relationship between the total cholesterol concentration in the sample solution and the response value of the current.

As is evident from the graph, according to the biosensor in accordance with the present invention, a favorable linearity between the total cholesterol concentration and the response current value can be obtained.

### Example 4

In the present example, a biosensor was produced which had the structures in accordance with Embodiment 3 shown in FIGS. 18 to 21 and whose object to be measured was glucose. Further, the reaction layer 218a included the electron mediator and glucose oxidase. It is to be noted that the reaction layer 218b was not formed in the case of glucose sensor.

First, the CMC layer 224 and the hydrophilic layer 225 were formed in the same manner as in Example 3. Further, 4 µl of an aqueous solution including 50 mM of potassium ferricyanide and 250 U/ml of glucose oxidase was dropped onto the CMC layer 224, which was then dried in a drying apparatus with warm blast at 50°C for 10 minutes to form the reaction layer 218a.

Thereafter, a biosensor in accordance with the present invention, whose object to be measured was glucose, was produced in the same manner as in Example 3.

### [Evaluation]

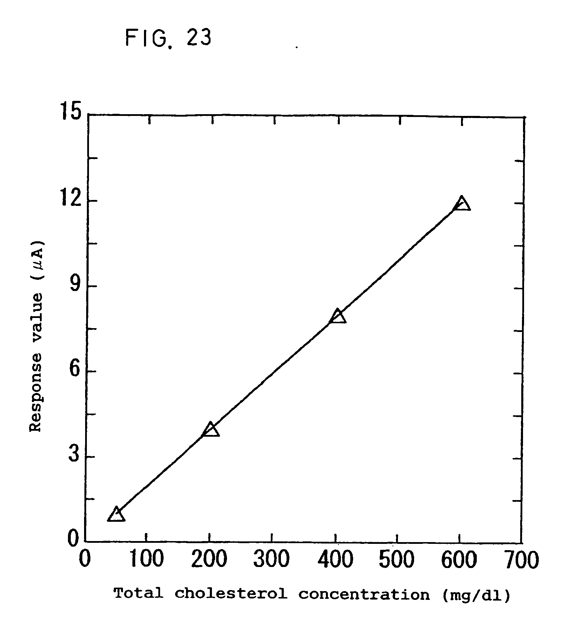

In the obtained biosensor, 5 µl of whole blood as the sample solution was added to the aperture 223 to serve as the sample solution adding part, 25 seconds layer, a pulse voltage of +0.2 V was applied to the measuring electrode toward the anode relative to the counter electrode, and five seconds later, a value of a current flowing between the working electrode and the counter electrode was measured. The results were shown in FIG. 23. FIG. 23 is a graph showing the relationship between the glucose concentration in the sample solution and the response value of the current.

As is obvious from FIG. 23, according to the biosensor in accordance with the present invention, a favorable linearity between the glucose concentration and the response current value can be obtained.

### Example 5

In the present example, a biosensor for measuring total cholesterol, having the structures in accordance with Embodiment 4 shown in FIGS. 24 to 27 was produced. The reaction layer 318a was added with the electron mediator and the reaction layer 318b was added with cholesterol oxidase, cholesterol esterase and a surfactant.

First, 5 µl of an aqueous solution containing 0.5 wt% of CMC was dropped onto the electrode system of the insulating base plate 301, and dried in a drying apparatus with warm blast at 50°C for 10 minutes to form the hydrophilic polymer layer 324 including CMC.

Next, 4 µl of a potassium ferricyanide aqueous solution (corresponding to 70 mM of potassium ferricyanide) was dropped onto the CMC layer 324, and dried in the drying apparatus with warm blast at 50°C for 10 minutes to form the reaction layer 318a including potassium ferricyanide.

Polyoxyethylene (10) octyl phenyl ether shown as TritonX-100 as the surfactant was added to an aqueous solution with cholesterol oxidase (EC1.1.3.6 : ChOD) originating from Nocardia and cholesterol esterase (EC.3.1.1.13 : ChE) originating from Pseudomonas dissolved therein. 0.4 µl of this mixed solution was dropped onto the concave part (slit 308, namely the sample solution supply pathway 308') of a member G obtained by integrating the cover 309 with the spacer 305, prefrozen with liquid nitrogen at -196°C, and dried in a freeze-drying apparatus for two hours. Thereby, the reaction layer 318b including 450 U/ml of cholesterol oxidase, 1,125 U/ml of cholesterol esterase and 2 wt% of the surfactant was formed.

The first filter was produced by cutting out Cyclopore membrane (pore size 5.0 *µ*m, thickness 8 to 23 *µ*m, manufactured by Whatman Plc.) in square shape. Further, the second filter was produced by cutting out Rapid 24 (maximum pore size 22 µm, thickness about 340 µm, manufactured by Whatman Plc.) in circular shape.

Thereafter, a member obtained by integrating to combine the insulating base plate 301, the combined member G and the second filter 326 was bonded to a member H obtained by integrating to combine the spacer 327, the first filter 304 and the sample solution dropping part 322, to produce a cholesterol sensor having the structures shown in FIGS. 24 to 27.

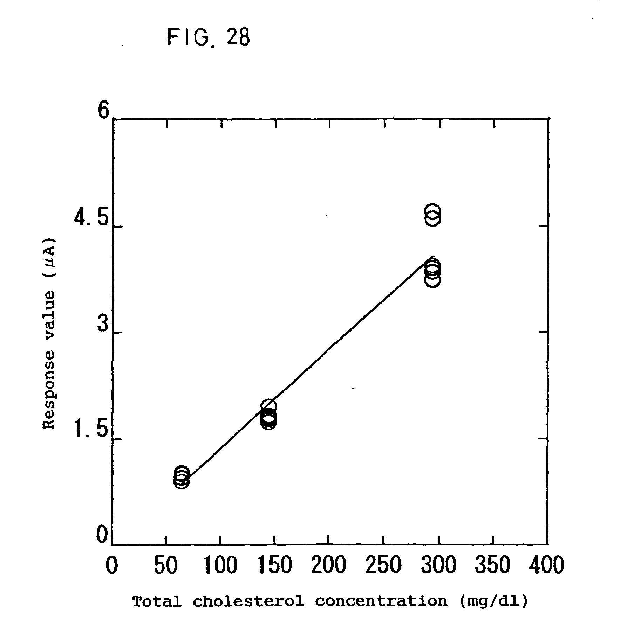

10 µl of whole blood as the sample solution was added to the sample solution dropping part of the biosensor as thus produced, 180 seconds layer, a pulse voltage of +0.2 V was applied to the measuring electrode toward the anode relative to the counter electrode, and five seconds later, a value of a current flowing between the working electrode and the counter electrode was measured. The results were shown in FIG. 28. FIG. 28 is a graph showing the relationship between the response value of the total cholesterol measurement sensor and the total cholesterol concentration. The measurement value (total cholesterol concentration) by Fuji Dry Chem. is plotted as abscissa, and the response current value in the present example as ordinate.

It was found from these results that the concentration measurement by the electrode system is possible by dropping of the sample solution onto the biosensor without pretreatment thereof, and a favorable linearity between the cholesterol concentration and the response value can be obtained.

### Example 6

In the present example, a biosensor was produced which had the structures in accordance with Embodiment 4 shown in FIGS. 24 to 27 and whose object to be measured was LDL cholesterol. The reaction layer 318a included the electron mediator and the reaction layer 318b included cholesterol oxidase, cholesterol esterase and a surfactant. Except for making the second filter 326 carry a substance capable of condensing lipoprotein except LDL, especially HDL, a biosensor was produced and then measurements were conducted in the same manner as in Example 5.

The substance for condensing or absorbing HDL may be exemplified by porous silica and an antibody against HDL.

Herein, an aqueous solution of the antibody against HDL and bovine serum albumin was dropped onto the second filter 326, prefrozen with liquid nitrogen at -196°C, and dried in a freeze-drying apparatus for two hours. As in FIG. 24, a biosensor was then produced which incorporated thereinto the second filter capable of capturing lipoprotein except LDL, especially HDL, in plasma.

### Example 7

In the present example, a biosensor was produced which had the structures in accordance with Embodiment 4 shown in FIGS. 24 to 27 and whose object to be measured was HDL cholesterol, and the reaction layer 318a included the electron mediator and the reaction layer 318b included cholesterol oxidase, cholesterol esterase and a surfactant. Further, the second filter 326 included a substance capable of condensing lipoprotein except HDL.

In the present example, except for making the second filter 326 carry a substance capable of condensing lipoproteins except HDL, a biosensor was produced in the same manner as in Example 1. Specifically, 2 mM of magnesium chloride and 50 mM of phosphotungstic acid, as substances capable of condensing lipoprotein except HDL, were dropped onto the second filter 326, prefrozen with liquid nitrogen at -196°C, and dried in a freeze-drying apparatus for two hours. As in FIG. 24, a biosensor was then produced which incorporated thereinto the second filter 326 capable of capturing lipoprotein except HDL.

### Industrial Applicability

In the biosensor in accordance with the present invention, filtration is conducted in a vertical direction by utilizing gravity and, further, a sample solution is moved to a measuring part such as an electrode by utilizing capillary action. To this end, the sample solution can be rapidly provided without application of pressure or the like, enabling rapid measurement. Since there is no fear of generation of bubbles in the sample solution supply pathway, highly accurate measurement is possible. Further, even in the case of realizing a configuration in which a measuring reagent is carried in position apart from the electrode, the filter and the measuring reagent can be separately incorporated into the biosensor of the present invention and the biosensor with such a configuration can be produced relatively easily in the production process.

Further, since the first filter has a space not in contact with the filter holding part, hemocytes in blood will not get through the part in contact with the filter holding part to be filtered and hence they will not be supplied to the sample solution supply pathway, whereby a biosensor with little variation can be obtained.

Moreover, in the first filter, the cross sectional area (F1) of the sample solution supply part side is larger than the cross sectional area (S1) of the sample solution supply pathway side so that an effect that plasma is rapidly provided into the biosensor can be obtained. Further, the cross sectional area (F1) of the sample solution supply part side of the first filter is larger than the cross sectional area (F2) of the portion, which is in contact with the base plate, of the first filter so that an effect that plasma is rapidly supplied into the biosensor can be obtained.

Additionally, with the use of a membrane filter, which will not substantially expand, for the biosensor of the present invention, hemocyte components in blood can be sufficiently filtered to measure total cholesterol in a highly accurate manner. Further, when reduction in sample amount is required, the use of the membrane filter is effective because of no substantial expansion thereof. Moreover, since the provision of the hydrophilic layer between the first filter and the base plate enables rapid supply of the sample solution having passed through the first filter to the sample solution supply pathway, an attempt can be made to shorten the measurement time.

Further, since the provision of the second filter between the first filter and the sample solution supply pathway allows rapid supply of the sample solution having passed through the first filter to the sample solution supply pathway, an attempt can be made to shorten the measurement time.

## Claims

1. A biosensor comprising: an insulating base plate (1, 101, 201, 301); an electrode system having a working electrode (2, 102, 202, 302) and a counter electrode (3, 103, 203, 303) which are provided on said base plate (1, 101, 201, 301); a reaction layer (18a, 18b, 118a, 118b, 218a, 218b, 318a, 318b) including at least oxidoreductase and an electron mediator; a sample solution supply pathway (8', 108', 208', 308') which includes said electrode system and said reaction layer (18a, 18b, 118a, 118b, 218a, 218b, 318a, 318b) and has an inlet (7, 107, 207, 307) (7, 107, 207, 307) and an air aperture 11, 111, 211a, 211b, 311a, 311b); a sample solution supply part for introducing a sample solution, which is in position apart from said sample solution supply pathway (8', 108', 208', 308'); and a first filter (4, 104, 204, 304) which is disposed between said sample solution supply pathway (8', 108', 208', 308') and said sample solution supply part for filtering said sample solution, where a filtrate filtered with said first filter (4, 104, 204, 304) is supplied into said sample solution supply pathway (8', 108', 208', 308') due to capillary action,
wherein the direction in which said sample solution passes through said first filter (4, 104, 204, 304) and the direction in which said filtrate passes through said sample solution supply pathway (8', 108', 208', 308') cross at right angles,
**characterised in that** said first filter (4, 104, 204, 304) does not intrude into said sample solution supply pathway (8', 108', 208', 308').

2. The biosensor in accordance with Claim 1, wherein said first filter (4, 104, 204, 304) is constituted by either a glass fiber or a cellulose fiber.

3. The biosensor in accordance with any of Claims 1 or 2, wherein said first filter (4, 104, 204, 304) is not in contact with said electrode system.

4. The biosensor in accordance with any of Claims 1 to 3, wherein at least part of said first filter (4, 104, 204, 304) is in contact with said insulating base plate (1, 101, 201, 301).

5. The biosensor in accordance with any of Claims 1 to 4, wherein said first filter (4, 104, 204, 304) is coated with any of polyvinyl alcohol, ethyl cellulose, hydroxypropyl cellulose, carboxymethyl cellulose, polyvinyl pyrrolidone, gelatin, agarose, polyacrylic acid and the salts thereof, starch and the derivatives thereof, polymers of maleic anhydride and the salts thereof, polyacrylamide, methacrylate resin, and poly-2-hydroxyethyl methacrylate.

6. The biosensor in accordance with any of Claims 1 to 5, wherein said reaction layer (18a, 18b, 118a, 118b, 218a, 218b, 318a, 318b) includes a reagent system for detecting total cholesterol.

7. The biosensor in accordance with any of Claims 1 to 6, wherein said first filter (4, 104, 204, 304) is constituted by a membrane filter with a uniform pore size in the form of a membrane.

8. The biosensor in accordance with Claim 7, wherein a hydrophilic layer is provided between said first filter (4, 104, 204, 304) and said base plate.

9. The biosensor in accordance with any of Claims 1 to 8, further comprising a second filter (326) between said first filter (4, 104, 204, 304) and said sample solution supply pathway.

10. The biosensor in accordance with Claim 9, wherein the mean pore size of said first filter (4, 104, 204, 304) is smaller than the mean pore size of said second filter.

11. The biosensor in accordance with Claim 9 or 10, wherein said second filter (326) is a depth filter.

12. The biosensor in accordance with any of Claims 9 to 11, wherein said first filter (4, 104, 204, 304) is in contact with said second filter.

13. The biosensor in accordance with any of Claims 9 to 12, wherein said second filter (326) is in contact with the inlet (7, 107, 207, 307) of said sample solution supply pathway.

14. The biosensor in accordance with any of Claims 9 to 13, wherein said second filter (326) is in not in contact with said electrode system.

15. The biosensor in accordance with any of Claims 9 to 14, wherein said first filter (4, 104, 204, 304) and/or said second filter (326) contain a reagent for suppressing a reaction between said oxidoreductase and cholesterol contained in any of high density lipoprotein, low density lipoprotein and very low density lipoprotein in said sample solution.

## Patentansprüche

1. Biosensor mit: einer isolierenden Grundplatte (1, 101, 201, 301); einem Elektrodensystem mit einer Arbeitselektrode (2, 102, 202, 302) und einer Gegenelektrode (3, 103, 203, 303), welche auf der Grundplatte (1, 101, 201, 301) vorgesehen sind; einer Reaktionsschicht (18a, 18b, 118a, 118b, 218a, 218b, 318a, 318b) einschließlich wenigstens einer Oxidoreduktase und einem Elektronenvermittler; einem Probenlösungszufuhrweg (8', 108', 208', 308'), welcher das Elektrodensystem und die Reaktionsschicht (18a, 18b, 118a, 118b, 218a, 218b, 318a, 318b) enthält und einen Einlass (7, 107, 207, 307) und eine Luftöffnung (11, 111, 211a, 211b, 311a, 311b) hat; einem Probenlösungszufuhrteil für das Einbringen einer Probenlösung, welches in einer Position abseits von dem Probenlösungszufuhrweg (8', 108', 208', 308') ist; und einem ersten Filter (4, 104, 204, 304), welcher zwischen dem Probenlösungszufuhrweg (8', 108', 208', 308') und dem Probenlösungszufuhrteil zum Filtrieren der Probenlösung angeordnet ist, wo ein mit dem ersten Filter (4, 104, 204, 304) filtriertes Filtrat in den Probenlösungszufuhrweg (8', 108', 208', 308') aufgrund der Kapillarwirkung zugeführt wird,
wobei die Richtung, in welcher die Probenlösung durch den ersten Filter (4, 104, 204, 304) tritt und die Richtung, in welcher das Filtrat durch den Probenlösungszufuhrweg (8', 108', 208', 308') tritt, sich im rechten Winkel überschneiden,
**dadurch gekennzeichnet, dass** der erste Filter (4, 104, 204, 304) nicht in den Probenlösungszufuhrweg (8', 108', 208', 308') eindringt.

2. Biosensor nach Anspruch 1, wobei der erste Filter (4, 104, 204, 304) entweder aus einer Glasfaser oder einer Cellulosefaser aufgebaut ist.

3. Biosensor nach einem der Ansprüche 1 oder 2, wobei der erste Filter (4, 104, 204, 304) nicht in Kontakt mit dem Elektrodensystem ist.

4. Biosensor nach einem der Ansprüche 1 bis 3, wobei wenigstens ein Teil des ersten Filters (4, 104, 204, 304) in Kontakt mit der isolierenden Grundplatte (1, 101, 201, 301) ist.

5. Biosensor nach einem der Ansprüche 1 bis 4, wobei der erste Filter (4, 104, 204, 304) mit einem Polyvinylalkohol, Ethylcellulose, Hydroxypropylcellulose, Carboxymethylcellulose, Polyvinylpyrrolidon, Gelatine, Agarose, Polyacrylsäure und den Salzen davon, Stärke und den Derivaten davon, Polymere von Maleinsäureanhydrid und den Salzen davon, Polyacrylamid, Methacrylatharz, oder Poly-2-hydroxyethylmethacrylat beschichtet ist.

6. Biosensor nach einem der Ansprüche 1 bis 5, wobei die Reaktionsschicht (18a, 18b, 118a, 118b, 218a, 218b, 318a, 318b) ein Reagenziensystem für den Nachweis des Gesamtcholesterins enthält.

7. Biosensor nach einem der Ansprüche 1 bis 6, wobei der erste Filter (4, 104, 204, 304) aus einem Membranfilter mit einer gleichmäßigen Porengröße in der Form einer Membran aufgebaut ist.

8. Biosensor nach Anspruch 7, wobei eine hydrophile Schicht zwischen dem ersten Filter (4, 104, 204, 304) und der Grundplatte vorgesehen ist.

9. Biosensor nach einem der Ansprüche 1 bis 8, der ferner einen zweiten Filter (326) zwischen dem ersten Filter (4, 104, 204, 304) und dem Probenlösungszufuhrweg umfasst.

10. Biosensor nach Anspruch 9, wobei die mittlere Porengröße des ersten Filters (4, 104, 204, 304) kleiner als die mittlere Porengröße des zweiten Filters ist.

11. Biosensor nach Anspruch 9 oder 10, wobei der zweite Filter (326) ein Tiefenfilter ist.

12. Biosensor nach einem der Ansprüche 9 bis 11, wobei der erste Filter (4, 104, 204, 304) in Kontakt mit dem zweiten Filter ist.

13. Biosensor nach einem der Ansprüche 9 bis 12, wobei der zweite Filter (326) in Kontakt mit dem Einlass (7, 107, 207, 307) des Probenlösungszufuhrweges ist.

14. Biosensor nach einem der Ansprüche 9 bis 13, wobei der zweite Filter (326) nicht in Kontakt mit dem Elektrodensystem ist.

15. Biosensor nach einem der Ansprüche 9 bis 14, wobei der erste Filter (4, 104, 204, 304) und/oder der zweite Filter (326) ein Reagenz enthält für die Unterdrückung einer Reaktion zwischen der Oxidoreduktase und dem Cholesterin, das in einem Lipoprotein mit hoher Dichte, Lipoprotein mit geringer Dichte und Lipoprotein mit sehr geringer Dichte in der Probenlösung enthalten ist.

## Revendications

1. Biocapteur comprenant : une plaque de base isolante (1, 101, 201, 301) ; un système d'électrodes comportant une électrode de travail (2, 102, 202, 302) et une contre-électrode (3, 103, 203, 303) qui sont prévues sur ladite plaque de base (1, 101, 201, 301) ; une couche de réaction (18a, 18b, 118a, 118b, 218a, 218b, 318a, 318b) incluant au moins une oxydoréductase et un médiateur d'électrons; une voie d'alimentation en solution échantillon (8', 108', 208', 308') qui inclut ledit système d'électrodes et ladite couche de réaction (18a, 18b, 118a, 118b, 218a, 218b, 318a, 318b) et comporte une entrée (7, 107, 207, 307) (7, 107, 207, 307) et une ouverture à l'air (11, 111, 211a, 211b, 311a, 311b) ; une partie d'alimentation en solution échantillon pour introduire une solution échantillon, qui se trouve dans une position distante de ladite voie d'alimentation en solution échantillon (8', 108', 208', 308') ; et un premier filtre (4, 104, 204, 304) qui est disposé entre ladite voie d'alimentation en solution échantillon (8', 108', 208', 308') et ladite partie d'alimentation en solution échantillon pour filtrer ladite solution échantillon, où un substrat filtré à l'aide dudit premier filtre (4, 104, 204, 304) est fourni dans ladite voie d'alimentation en solution échantillon (8', 108', 208', 308') du fait de l'action capillaire,
dans lequel la direction dans laquelle ladite solution échantillon passe à travers le premier filtre (4, 104, 204, 304) et la direction dans laquelle ledit filtrat passe à travers ladite voie d'alimentation en solution échantillon (8', 108', 208', 308') se croisent à angle droit,
**caractérisé en ce que** ledit premier filtre (4, 104, 204, 304) ne s'introduit pas dans ladite voie d'alimentation en solution échantillon (8', 108', 208', 308').

2. Biocapteur selon la revendication 1, dans lequel ledit premier filtre (4, 104, 204, 304) est constitué soit par une fibre de verre, soit par une fibre de cellulose.

3. Biocapteur selon l'une quelconque des revendications 1 ou 2, dans lequel ledit premier filtre (4, 104, 204, 304) n'est pas en contact avec le système d'électrodes.

4. Biocapteur selon l'une quelconque des revendications 1 à 3, dans lequel au moins une partie dudit premier filtre (4, 104, 204, 304) est en contact avec la plaque de base isolante (1, 101, 201, 301).

5. Biocapteur selon l'une quelconque des revendications 1 à 4, dans lequel ledit premier filtre (4, 104, 204, 304) est revêtu de l'un quelconque d'un alcool polyvinylique, d'une éthyl cellulose, d'une hydroxypropyl cellulose, d'une carboxyméthyl cellulose, d'une polyvinyl pyrrolidone, d'une gélatine, d'agarose, d'un acide polyacrylique et les sels de celui-ci, d'amidon et les dérivés de celui-ci, de polymères de l'anhydride maléique et les sels de ceux-ci, d'un polyacrylamide, d'une résine de méthacrylate, et d'un méthacrylate de poly-2-hydroxyéthyle.

6. Biocapteur selon l'une quelconque des revendications 1 à 5, dans lequel ladite couche de réaction (18a, 18b, 118a, 118b, 218a, 218b, 318a, 318b) inclut un système de réactifs destiné à détecter le cholestérol total.

7. Biocapteur selon l'une quelconque des revendications 1 à 6, dans lequel ledit premier filtre (4, 104, 204, 304) est constitué d'un filtre à membrane doté d'une taille de pores uniforme sous la forme d'une membrane.

8. Biocapteur selon la revendication 7, dans lequel une couche hydrophile est prévue entre ledit premier filtre (4, 104, 204, 304) et ladite plaque de base.

9. Biocapteur selon l'une quelconque des revendications 1 à 8, comprenant en outre un second filtre (326) entre ledit premier filtre (4, 104, 204, 304) et ladite voie d'alimentation en solution échantillon.

10. Biocapteur selon la revendication 9, dans lequel la taille de pores moyenne dudit premier filtre (4, 104, 204, 304) est plus petite que la taille de pores moyenne dudit second filtre.

11. Biocapteur selon la revendication 9 ou 10, dans lequel ledit second filtre (326) est un filtre en profondeur.

12. Biocapteur selon l'une quelconque des revendications 9 à 11, dans lequel ledit premier filtre (4, 104, 204, 304) est en contact avec ledit second filtre.

13. Biocapteur selon l'une quelconque des revendications 9 à 12, dans lequel ledit second filtre (326) est en contact avec l'entrée (7, 107, 207, 307) de ladite voie d'alimentation en solution échantillon.

14. Biocapteur selon l'une quelconque des revendications 9 à 13, dans lequel ledit second filtre (326) n'est pas en contact avec ledit système d'électrodes.

15. Biocapteur selon l'une quelconque des revendications 9 à 14, dans lequel ledit premier filtre (4, 104, 204, 304) et/ou ledit second filtre (326) contiennent un réactif destiné à supprimer une réaction entre ladite oxydoréductase et le cholestérol contenu dans l'une quelconque d'une lipoprotéine de haute densité, d'une lipoprotéine de basse densité et d'une lipoprotéine de très basse densité se trouvant dans ladite solution échantillon.
